(19) Europäisches Patentamt
European Patent Office
Office européen des brevets

(11) EP 4 122 462 A1

(12) **EUROPEAN PATENT APPLICATION**
published in accordance with Art. 153(4) EPC

(43) Date of publication:
**25.01.2023 Bulletin 2023/04**

(21) Application number: **21771821.2**

(22) Date of filing: **19.03.2021**

(51) International Patent Classification (IPC):
***A61K 31/365*** *(2006.01)* ***A61P 25/02*** *(2006.01)*
***A61P 35/00*** *(2006.01)*

(52) Cooperative Patent Classification (CPC):
**A61K 31/365; A61P 25/02; A61P 35/00**

(86) International application number:
**PCT/CN2021/081817**

(87) International publication number:
**WO 2021/185356 (23.09.2021 Gazette 2021/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.03.2020 CN 202010202588**
**07.08.2020 CN 202010790778**
**06.11.2020 CN 202011233563**

(71) Applicant: **CSPC NBP PHARMACEUTICAL CO., LTD.**
**Shijiazhuang, Hebei 052165 (CN)**

(72) Inventors:
- **LIU, Xibao**
  **Shijiazhuang, Hebei 052165 (CN)**
- **LI, Yanling**
  **Shijiazhuang, Hebei 052165 (CN)**
- **LIU, Jieru**
  **Shijiazhuang, Hebei 052165 (CN)**
- **APFEL, Stuart C.**
  **Shijiazhuang, Hebei 052165 (CN)**
- **MA, Yuxiu**
  **Shijiazhuang, Hebei 052165 (CN)**
- **WU, Xiaojuan**
  **Shijiazhuang, Hebei 052165 (CN)**
- **WANG, Yuqing**
  **Shijiazhuang, Hebei 052165 (CN)**
- **YANG, Hanyu**
  **Shijiazhuang, Hebei 052165 (CN)**

(74) Representative: **Mewburn Ellis LLP**
**Aurora Building**
**Counterslip**
**Bristol BS1 6BX (GB)**

# (54) USE OF BUTYLPHTHALIDE AND DERIVATIVE THEREOF

(57) The present application provides application of butylphthalide or optical isomer, prodrug, deuterium, metabolite, and ring-opening product or ring-opening product salt thereof in the preparation of a drug for preventing, alleviating, or treating peripheral neuropathy, especially chemotherapy-induced peripheral neuropathy, and a use method of butylphthalide and a derivative thereof in preventing or treating peripheral neuropathy, especially chemotherapy-induced peripheral neuropathy. In vivo and in vitro studies show that the drug of the present invention can effectively prevent, alleviate, or treat peripheral neuropathy caused by chemotherapy drugs without affecting the tumor-inhibiting efficacy and pharmacokinetic properties of the chemotherapy drugs.

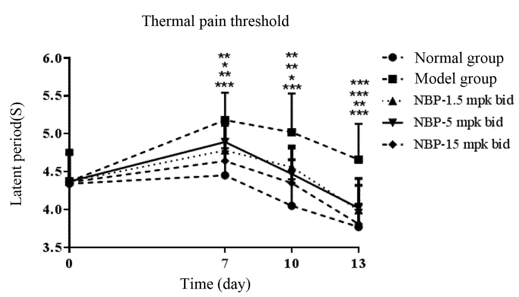

Fig. 3

EP 4 122 462 A1

**Description**

CROSS-REFERENCE TO RELATED APPLICATIONS

The present disclosure claims priority and rights to three Chinese invention applications submitted to the China National Intellectual Property Administration as follows: a prior application No. 202010202588.4 titled "USE OF BUTYLPHTHALIDE AND DERIVATIVE THEREOF" and filed on 20 March 2020, a prior application No. 202010790778.2 titled "USE OF BUTYLPHTHALIDE AND DERIVATIVE THEREOF" and filed on 7 August 2020, and a prior application No. 202011233563.7 titled "USE OF BUTYLPHTHALIDE AND DERIVATIVE THEREOF" and filed on 6 November 2020. The three prior applications are entirely incorporated herein by reference.

TECHNICAL FIELD

The present disclosure refers to the pharmaceutical field, and specifically refers to use of butylphthalide and a derivative thereof in the preparation of a drug for preventing, relieving, or treating peripheral neuropathy, and particularly in the preparation of a drug for preventing, relieving, or treating chemotherapy-induced peripheral neuropathy, as well as a method for preventing, relieving, or treating peripheral neuropathy, and particularly for preventing, relieving, or treating chemotherapy-induced peripheral neuropathy by using butylphthalide and a derivative thereof.

BACKGROUND

Peripheral neuropathy is a syndrome consisting of sensory loss, muscle weakness and atrophy, tendon hyporeflexia, and vasomotion symptom alone or in any combination. There are many clinical peripheral neuropathies, including metabolic neuropathy such as diabetic neuropathy; traumatic neuropathy such as carpal tunnel syndrome; immune neuropathy, and the like. Chemotherapy-induced peripheral neuropathy (CIPN) is a common dose-limiting adverse reaction during chemotherapy of a cancer patient. The CIPN may be the most common type of toxic neuropathy, affecting between 58% and 78% of patients who receive neurotoxic chemotherapies (Seretny M, Currie GL, Sena ES, et al. (2014). Incidence, prevalence, and predictors of chemotherapy-induced peripheral neuropathy: a systemic review and meta-analysis. Pain 155: 2461-2470), and has particularly important clinical consequences. In most instances, it is the dose-limiting toxicity that restricts the use of chemotherapeutic agents in the treatment of malignant tumors. The presence of toxic neuropathy often leads to dose reduction, and sometimes results in the discontinuation of potentially life-saving therapy (Gadgil S, Ergun M, van den Heuval SA, et al. (2019) A systematic summary and comparison of animal models for chemotherapy induced peripheral neuropathy (CIPN). PLOS ONE https://doi.org/10.1371/journal.pone.0221787). Among patients who are administered potentially neurotoxic chemotherapeutic agents, it is estimated that by 6 months following the start of treatment, 30% to 71% continue to experience symptoms related to CIPN.

The CIPN is mainly induced by a chemotherapeutic drug, such as platinum (e.g., cisplatin, carboplatin, or oxaliplatin), paclitaxels (e.g., paclitaxel or docetaxel), vinca alkaloids (e.g., vincristine or vinblastine), a protease inhibitor (e.g., bortezomib), immunomodulator (thalidomide or lenalidomide), or epothilone (e.g., ixabepilone). The most common symptoms with CIPN are paresthesias of hands and feet, such as: pain (burning pain, sharp shooting pains, dysesthesias, allodynia, and hyperalgesia), tingling (paresthesias), and numbness (Ewertz M, Qvortrup C, Eckhoff L. (2015) Chemotherapy-induced peripheral neuropathy in patients treated with taxanes and platinum derivatives. ActaOncologica 54: 587-591); other common clinical findings include diminished or absent deep tendon reflexes, loss of temperature or vibratory sensation, orthostatic hypotension, and sensory ataxia (especially with the platinum based drugs). Less frequent are motion symptoms (e.g., distal or proximal weakness, muscle cramps and gait dysfunction), as well as autonomic symptoms (e.g., constipation or diarrhea, sweating, and lightheadedness with positional changes).

The clinical course of CIPN varies with different individual chemotherapeutic agents. With most chemotherapeutic agent symptoms of neuropathy begin to appear after a cumulative threshold dose has been reached and continue to progress with additional dosing. Depending on the specific agent, adjusting the dosing regimen, the frequency of dosing, and even the rate of infusion may impact the frequency of neuropathy that develops in a given population, but no adjustment to the regimen apart from discontinuing the chemotherapy will completely prevent CIPN in a treatment cohort. With some chemotherapeutic agents, symptoms will continue to progress for some time even after discontinuing the responsible agent, a phenomenon known as coasting. Even if the symptoms are relatively stable, they may persist for months, years, or indefinitely.

Two members of the taxane chemical family are commonly used therapeutically to treat a variety of cancers; paclitaxel and docetaxel. Taxanes, inhibit cancer growth by disassembling microtubules necessary for mitotic activity, and by the same mechanism disrupt microtubule based axonal transport, inhibiting trophic support for sensory neurons. Taxanes are commonly used to treat breast cancer, ovarian cancer, non-small cell lung cancer, gastric cancer, head and neck cancer and prostate cancer. Moderate or severe (grade 2-4) sensory neuropathy affecting both small diameter

and large fiber sensory neurons occurs in 20-35% of patients who receive paclitaxel at a cumulative dose of 715 -1500 mg/m2 or docetaxel at a cumulative dose of 371-600 $mg/m^2$. Sensory symptoms (numbness, tingling and pain) typically begin in the distal extremities, first the legs then the hands which is consistent with a mechanism of action targeting axonal transport. In 400 of these patients, symptoms may persist for 3 years or more (Park SB, Goldstein D, Krishnan AV, et al. (2013) Chemotherapy-induced peripheral neurotoxicity: A critical analysis. CA Cancer J Clin 63: 419-437), while 50% recover within 9 months. While sensory neuropathy is more common and more severe in patients with taxane neuropathy, some patients also develop a deficit in fine motor control.

Cisplatin, carboplatin and oxaliplatin are platinum based drugs used to treat cancer of the testes, ovary, cervix, head and neck, colon and rectum. Their mechanism of action is to cross link DNA limiting the ability of cancer cells to engage in DNA synthesis. When cumulative doses of 600 $mg/m^2$ or more are administered, it often results in a severe sensory ataxia secondary to injury of large diameter proprioceptive neurons. 50% who receive cumulative doses of 1170 $mg/m^2$ develop a debilitating grade 3 sensory neuropathy associated with degeneration of the sensory dorsal root ganglia. The most prominent findings are tingling, numbness, loss of proprioception and diminished or absent deep tendon reflexes, all indicative of predominantly large sensory fiber loss (Park SB, Goldstein D, Krishnan AV, et al. (2013) Chemotherapy-induced peripheral neurotoxicity: A critical analysis. CA Cancer J Clin 63: 419-437). Reduced sensory nerve conduction velocity and amplitudes are also seen. In addition to this chronic form of neuropathy, oxaliplatin is also associated with an acute type of neuropathy that immediately follows the infusion and affects the face in addition to the extremities. Coasting is common.

Vincristine and vinblastine are the most clinically important vinca alkaloids, and are used to treat leukemia, Hodgkin disease, malignant lymphoma, neuroblastoma, Wilms tumor, Kaposi sarcoma, melanoma, lung cancer, and uterine cancers. Their mechanism of action targets microtubule aggregation and results in a loss of cell division leading to cell death. 35% to 45% of patients treated with vincristine experience abnormal fine motor function and abnormal walking in addition to sensory deficits. 30% experience coasting when the chemotherapy is stopped (Park SB, Goldstein D, Krishnan AV, et al. (2013) Chemotherapy-induced peripheral neurotoxicity: A critical analysis. CA Cancer J Clin 63: 419-437).

Bortezomib is a relatively new chemotherapeutic agent that is highly effective at treating patients with multiple myeloma and some other hematological malignancies. Bortezomib administration results in a severe painful sensory neuropathy in 50% and about an additional 30% experience moderate to severe neurotoxicity. Symptoms include paresthesias and numbness in the distal extremities, especially in the lower limbs, sharp burning pain. The CIPN associated with Bortezomib is a predominantly small fiber sensory neuropathy, however, some patients also develop autonomic involvement and may present with orthostatic hypotension, suppressed heart rate variability, and delayed gastric emptying. Motor involvement is rare but does occur on occasion. The neuropathy has its onset with administration of cumulative doses of 16-26 $mg/m^2$, and increases in incidence with higher doses until a dose of 40-45 $mg/m^2$ is administered, when the incidence plateaus. The neuropathy is slow to recover, and 2-3 months after treatment 60-85% of patients still have clinically significant neuropathy.

Thalidomide and lenalidomide are synthetic glutamic acid derivatives used for treating relapsed and refractory multiple myeloma. CIPN is a major adverse reaction of this type of drug therapy, and the remission is slow or even irreversible. Thalidomide-induced neuropathy is mainly alliesthesia, which first occurs in the feet, then extends to the hands, often in a sock-like distribution, i.e., severe in the distal end without extending to above the knees and elbows. It includes hypoesthesia, hyperesthesia, disesthesia, muscle pain, haphalgesia, numbness, tingling, burning pain, tenseness, coldness in hands and feet, pallor, itchy legs, and anthurium (Bkl D, de Souza S M, Costa-Lima C, et al. Thalidomide for the treatment of gastrointestinal bleeding due to angiodysplasia in a patient with Glanzmann's thrombasthenia [J]. Hematol Rep, 2017, 9(2): 6961). Some literatures show that when the cumulative dose of thalidomide exceeds 20 g, there is a positive correlation between the occurrence and severity of neuropathy and the cumulative dose (Rui Gui et al., Medical Recapitulate, P1739-1742, Vol 18 No 11, June 2012).

Ixabepilone is a semi-synthetic epothilone lactam analog, and the first epothilone antitumor drug in the world. Compared with paclitaxel, the two drugs have different structures, but have a similar mechanism of action, i.e., causing tumor cell apoptosis by interfering with the cancer cell fission. Ixabepilone can induce peripheral lesions, causing abnormal sensory nerves. Such adverse reaction will generally disappear after 1 to 2 months of drug withdrawal. The degree of sensory neuropathy is related with the administration dose and the administration rate (Burotto M, Edgerly M, Velarde M, et al. A phase II multi-center study of bevacizumab in combination with ixabepilone in subjects with advanced renal cell carcinoma [J]. Oncologist, 2017, 22(8): 888-e84.)

Despite the fact that CIPN is considered as the most common severe side effect associated with chemotherapy, the American Society of Clinical Oncology clinical practice guidelines has concluded that there is currently no effective theatment available to prevent or reverse CIPN, and only recommends one drug (duloxetine) (see Table 1 (prevention) and Table 2 (treatment), Hershman DL, Lacchetti C, Dworkin RH, et al. (2014). Prevention and management of chemotherapy-induced peripheral neuropathy in survivors of adult cancers: American society of clinical oncology clinical practice guideline. J Clin Oncol 32:1941-67). The treatment is only limited to the treatment with drugs for neuropathic

pain, the supportive care, and the chemotherapeutic dose adjustment (Kolb N, and Burns T (2018) Clinical Research in chemotherapy-induced peripheral neuropathy. Neurology 91: 379-380). Therefore, effective prevention and treatment of CIPN is a clinical problem to be urgently solved.

Table 1

| Interventions | Strength of Recommendation | Strength of Evidence | Efficacy | Safety |
|---|---|---|---|---|
| Acetyl L-carnitine | Strong against | High | Low | Low |
| Diethyldithio-carbamate | Strong against | Low | No evidence | High |
| Nimodipine | Strong against | Low | No evidence | Moderate |
| Amifostine | Moderate against | Intermediate | Low | Moderate |
| Amitriptyline | Moderate against | Intermediate | No evidence | Moderate |
| Glutathione for patients receiving paclitaxel/carboplatin chemotherapy | Moderate against | Intermediate | Low | Low |
| Org2766 | Moderate against | Intermediate | Low | Low |
| All-trans retinoic acid | Moderate against | Low | Low | Moderate |
| rhuLIF | Moderate against | Low | No evidence | Low |
| Vitamin E | Moderate against | Intermediate | Low | Low |
| Calcium and magnesium for patients receiving oxaliplatin-based chemotherapy | Moderate against | Low | Low | Low |
| Glutathione for patients receiving cisplatin/ oxaliplatin-based chemotherapy | Inconclusive | Low | Low | Low |
| Acetylcysteine | Inconclusive | Low | Low | Low |
| Carbamazepine | Inconclusive | Low | Low | Low |
| Glutamate | Inconclusive | Low | Low | Low |
| Goshajinkigan | Inconclusive | Low | Low | Low |
| Omega-3-fatty acid for patients receiving oxaliplatin treatment | Inconclusive | Low | Low | Low |
| Venlafaxine | Insufficient | Low | Low | Low |

Table 2

| Interventions | Strength of Recommendation | Strength of Evidence | Efficacy | Safety |
|---|---|---|---|---|
| Duloxetine | Moderate for | Intermediate | Moderate | Low |
| Lamotrigine | Moderate against | Intermediate | No evidence | Low |
| Acetyl L-carnitine | Inconclusive | Low | Low | Moderate |
| Amitriptyline/nortriptyline | Inconclusive | Intermediate | Low | Low |
| Gabapentin | Inconclusive | Intermediate | Low | Low |
| Baclofen, amitriptyline, and ketamine gels for external use | Inconclusive | Intermediate | Moderate | Low |

Butylphthalide (3-n-butylphathlide, NBP) (chemical name: 3-n-butylphthalide, and trade name: NBP) has a structure as shown in formula (I), and is a drug for treating mild to moderate ischemic apoplexy.

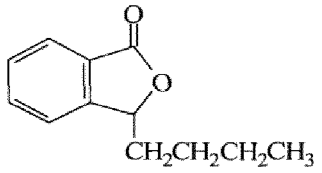

(I)

At present, no report is available on use of butylphthalide in the prevention or treatment of peripheral neuropathy, and particularly chemotherapy-induced peripheral neuropathy.

SUMMARY

An object of the present disclosure is to provide use of butylphthalide or an optical isomer, a prodrug, a deuterated product, a metabolite, a ring-opening product, or a salt of the ring-opening product thereof in the preparation of a drug for preventing, relieving, or treating peripheral neuropathy. The peripheral neuropathy is preferably a drug-induced peripheral neuropathy, and more preferably a chemotherapeutic drug-induced peripheral neuropathy. The preventing, relieving, or treating peripheral neuropathy includes, but is not limited to, preventing, relieving, or treating chemotherapeutic drug-induced paresthesia and dyskinesia, such as paresthesia of pain and heat, or impaired motor coordination.

In some embodiments, the drug is in a single dose form or a divided dose form.

In some embodiments, the drug is prepared into a clinically acceptable formulation, such as an oral formulation, an injectable formulation, a topical formulation, or an external formulation, and preferably an oral formulation and an injectable formulation.

In some embodiments, the drug is an oral formulation. The oral formulation comprises from about 1 mg to about 1,000 mg, preferably from about 1 mg to about 500 mg, or from about 1 mg to about 300 mg, or from about 1 mg to about 200 mg, or from about 5 mg to about 180 mg, or from about 10 mg to about 150 mg, or from about 30 mg to about 120 mg, or from about 50 mg to about 120 mg, or from about 80 mg to about 120 mg, or from about 90 mg to about 110 mg, or about 100 mg, of butylphthalide or the optical isomer, the prodrug, the deuterated product, the metabolite, the ring-opening product, or the salt of the ring-opening product thereof, on the basis of butylphthalide.

In some embodiments, a daily dose of the oral formulation is from about 1 mg to about 10,000 mg, preferably from about 10 mg to about 5,000 mg, or from about 20 mg to about 3,000 mg, or from about 30 mg to about 2,000 mg, or from about 50 mg to about 1,500 mg, or from about 70 mg to about 1,200mg, or from about 100 mg to about 1,000 mg, or from about 200 mg to about 900 mg, or from about 300 mg to about 800 mg, or from about 400 mg to about 700 mg, or from about 500 mg to about 600 mg, or from about 60 mg to about 800 mg, or from about 60 mg to about 600 mg, or from about 100 mg to about 800 mg, or from about 100 mg to about 600 mg, or from about 200 mg to about 600 mg, or from about 200 mg to about 800 mg, or from about 300 mg to about 600 mg, or from about 400 mg to about 600 mg, or from about 400 mg to about 800 mg, on the basis of butylphthalide.

In some embodiments, the oral formulation is administered once per day by administering from about 60 mg to about 800 mg of the oral formulation of butylphthalide or the optical isomer, the prodrug, the deuterated product, the metabolite, the ring-opening product, or the salt of the ring-opening product thereof each time, e.g., administering about 60 mg, about 100 mg, about 200 mg, about 300 mg, about 400 mg, about 500 mg, about 600 mg, about 700 mg, or about 800 mg of the oral formulation each time, on the basis of butylphthalide; or, the oral formulation is administered twice per day by administering from about 30 mg to about 400 mg of the oral formulation of butylphthalide or the optical isomer, the prodrug, the deuterated product, the metabolite, the ring-opening product, or the salt of the ring-opening product thereof each time, e.g., administering about 30 mg, about 50 mg, about 100 mg, about 150 mg, about 200 mg, about 250 mg, about 300 mg, about 350 mg, or about 400 mg of the oral formulation each time, on the basis of butylphthalide; or, the oral formulation is administered thrice per day by administering from about 20 mg to about 300 mg of the oral formulation of butylphthalide or the optical isomer, the prodrug, the deuterated product, the metabolite, the ring-opening product, or the salt of the ring-opening product thereof each time, e.g., administering about 20 mg, about 50 mg, about 100 mg, about 150 mg, about 200 mg, about 250 mg, or about 300 mg of the oral formulation each time, on the basis of butylphthalide.

In some embodiments, the drug is an injectable formulation. The injectable formulation comprises from about 0.001 mg/mL to about 100 mg/mL, preferably from about 0.005 mg/mL to about 50 mg/mL, or from about 0.01 mg/mL to about 10 mg/mL, or from about 0.1 mg/mL to about 5 mg/mL, or from about 0.1 mg/mL to about 3 mg/mL, or from about 0.1 mg/mL to about 1 mg/mL, or from about 0.12 mg/mL to about 0.80 mg/mL, or from about 0.15 mg/mL to about 0.50 mg/mL, more preferably from about 0.20 mg/mL to about 0.40 mg/mL, or from about 0.20 mg/mL to about 0.30 mg/mL, or about 0.25 mg/mL, of butylphthalide or the optical isomer, the prodrug, the deuterated product, the metabolite, the ring-opening product, or the salt of the ring-opening product thereof, on the basis of butylphthalide.

In some embodiments, a daily dose of the injectable formulation is from about 1 mg to about 1,000 mg, preferably

from about 5 mg to about 500 mg, or from about 10 mg to about 300 mg, or from about 15 mg to about 200 mg, or from about 20 mg to about 150 mg, or from about 25 mg to about 120 mg, or from about 30 mg to about 100 mg, or from about 35 mg to about 90 mg, or from about 40 mg to about 80 mg, or from about 45 mg to about 70 mg, or from about 50 mg to about 60 mg, or from about 1 mg to about 100 mg, or from about 2 mg to about 80 mg, or from about 5 mg to about 75 mg, or from about 10 mg to about 50 mg, or from about 15 mg to about 50 mg, or from about 20 mg to about 50 mg, or from about 25 mg to about 75 mg, or from about 25 mg to about 50 mg, on the basis of butylphthalide.

In some embodiments, the injectable formulation is administered once per day by administering from about 1 mg to about 100 mg, preferably about 1 mg, about 2 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg, of the injectable formulation of butylphthalide or the optical isomer, the prodrug, the deuterated product, the metabolite, the ring-opening product, or the salt of the ring-opening product thereof each time, on the basis of butylphthalide; or, the injectable formulation is administered twice per day by administering from about 1 mg to about 50 mg, preferably about 1 mg, about 2 mg, about 2.5 mg, about 5 mg, about 10 mg, about 15 mg, about 20 mg, about 25 mg, about 30 mg, about 35 mg, about 40 mg, about 45 mg, or about 50 mg, of butylphthalide or the optical isomer, the prodrug, the deuterated product, the metabolite, the ring-opening product, or the salt of the ring-opening product thereof each time.

In some embodiments, the chemotherapeutic drug includes, but is not limited to, one of or the combination of more of the followings: (1) a chemotherapeutic drug acting on a microtubule system or an anti-mitotic chemotherapeutic drug, including: a taxane drug, such as paclitaxel, docetaxel or the like; or a vinca alkaloid drug, such as vincristine, vinblastine or the like; or an epothilone drug, such as ixabepilone or the like; or a protease inhibitor drug, such as bortezomib or the like; or (2) a chemotherapeutic drug that interferes with DNA synthesis, including a platinum drug, such as cisplatin, carboplatin, oxaliplatin or the like; or (3) an immunomodulator drug, such as thalidomide, lenalidomide or the like.

In some embodiments, the butylphthalide or the optical isomer, the prodrug, the deuterated product, the metabolite, the ring-opening product, or the salt of the ring-opening product thereof may be used in the preparation of the drug in combination with one or more of other drugs for treating peripheral neuropathy, wherein the other drugs for treating peripheral neuropathy are preferably duloxetine or a salt thereof, or monosialotetrahexosyl ganglioside sodium.

Another object of the present disclosure further includes providing a method for preventing, relieving, or treating peripheral neuropathy, including administering to a subject or patient a therapeutically effective amount of butylphthalide or an optical isomer, a prodrug, a deuterated product, a metabolite, a ring-opening product, or a salt of the ring-opening product thereof, wherein the peripheral neuropathy is preferably a drug-induced peripheral neuropathy, more preferably a chemotherapeutic drug-induced peripheral neuropathy.

In some embodiments, the administration may be oral administration, injection administration, topical administration, or in vitro administration, preferably oral administration or injection administration. The therapeutically effective amount can prevent, treat, or relieve the peripheral neuropathy in the subject or patient.

In some embodiments, the administration may be oral administration. The therapeutically effective amount refers to a daily dose from about 1 mg to about 10,000 mg, preferably from about 10 mg to about 5,000 mg, or from about 20 mg to about 3,000 mg, or from about 30 mg to about 2,000 mg, or from about 50 mg to about 1,500 mg, or from about 70 mg to about 1,200mg, or from about 100 mg to about 1,000 mg, or from about 200 mg to about 900 mg, or from about 300 mg to about 800 mg, or from about 400 mg to about 700 mg, or from about 500 mg to about 600 mg, or from about 60 mg to about 800 mg, or from about 60 mg to about 600 mg, or from about 100 mg to about 800 mg, or from about 100 mg to about 600 mg, or from about 200 mg to about 600 mg, or from about 200 mg to about 800 mg, or from about 300 mg to about 600 mg, or from about 400 mg to about 600 mg, or from about 400 mg to about 800 mg, on the basis of butylphthalide.

In some embodiments, the administration may be injection administration. The therapeutically effective amount refers to a daily dose from about 1 mg to about 1,000 mg, preferably from about 5 mg to about 500 mg, or from about 10 mg to about 300 mg, or from about 15 mg to about 200 mg, or from about 20 mg to about 150 mg, or from about 25 mg to about 120 mg, or from about 30 mg to about 100 mg, or from about 35 mg to about 90 mg, or from about 40 mg to about 80 mg, or from about 45 mg to about 70 mg, or from about 50 mg to about 60 mg, or from about 1 mg to about 100 mg, or from about 2 mg to about 80 mg, or from about 5 mg to about 75 mg, or from about 10 mg to about 50 mg, or from about 15 mg to about 50 mg, or from about 20 mg to about 50 mg, or from about 25 mg to about 75 mg, or from about 25 mg to about 50 mg, on the basis of butylphthalide.

In some embodiments, the butylphthalide or the optical isomer, the prodrug, the deuterated product, the metabolite, the ring-opening product, or the salt of the ring-opening product thereof is used in a single dose form or a divided dose form.

In some embodiments, the method for preventing, relieving, or treating comprising administration once per day by administering from about 60 mg to about 800 mg of the oral formulation of butylphthalide or the optical isomer, the prodrug, the deuterated product, the metabolite, the ring-opening product, or the salt of the ring-opening product thereof

each time, e.g., administering about 60 mg, about 100 mg, about 200 mg, about 300 mg, about 400 mg, about 500 mg, about 600 mg, about 700 mg, or about 800 mg of the oral formulation each time, on the basis of butylphthalide; or, administration twice per day by administering from about 30 mg to about 400 mg of the oral formulation of butylphthalide or the optical isomer, the prodrug, the deuterated product, the metabolite, the ring-opening product, or the salt of the ring-opening product thereof each time, e.g., administering about 30 mg, about 50 mg, about 100 mg, about 150 mg, about 200 mg, about 250 mg, about 300 mg, about 350 mg, or about 400 mg of the oral formulation each time, on the basis of butylphthalide; or, administration thrice per day by administering from about 20 mg to about 300 mg of the oral formulation of butylphthalide or the optical isomer, the prodrug, the deuterated product, the metabolite, the ring-opening product, or the salt of the ring-opening product thereof each time, e.g., administering about 20 mg, about 50 mg, about 100 mg, about 150 mg, about 200 mg, about 250 mg, or about 300 mg of the oral formulation each time, on the basis of butylphthalide; or administration once per day by administering from about 1 mg to about 100 mg, preferably about 1 mg, about 2 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg, of the injection formulation of butylphthalide or the optical isomer, the prodrug, the deuterated product, the metabolite, the ring-opening product, or the salt of the ring-opening product thereof each time, on basis of butylphthalide; or, administration twice per day by administering from about 1 mg to about 50 mg, preferably about 1 mg, about 2 mg, about 2.5 mg, about 5 mg, about 10 mg, about 15 mg, about 20 mg, about 25 mg, about 30 mg, about 35 mg, about 40 mg, about 45 mg, or about 50 mg, of the injection formulation of butylphthalide or the optical isomer, the prodrug, the deuterated product, the metabolite, the ring-opening product, or the salt of the ring-opening product thereof each time, on the basis of butylphthalide.

In some embodiments, the chemotherapeutic drug includes, but is not limited to, one or the combination of more of the followings: (1) a chemotherapeutic drug acting on a microtubule system or an anti-mitotic chemotherapeutic drug, including: a taxane drug, such as paclitaxel, docetaxel or the like; or a vinca alkaloid drug, such as vincristine, vinblastine or the like; or an epothilone drug, such as ixabepilone or the like; or a protease inhibitor drug, such as bortezomib or the like; or (2) a chemotherapeutic drug that interferes with DNA synthesis, including a platinum drug, such as cisplatin, carboplatin, oxaliplatin or the like; or (3) an immunomodulator drug, such as thalidomide, lenalidomide or the like.

In some embodiments, the butylphthalide or the optical isomer, the prodrug, the deuterated product, the metabolite, the ring-opening product, or the salt of the ring-opening product thereof may be used in combination with one or more of other drugs for treating peripheral neuropathy, wherein the other drugs for treating peripheral neuropathy are preferably duloxetine or a salt thereof, monosialotetrahexosyl ganglioside sodium.

Another object of the present disclosure further includes providing butylphthalide or an optical isomer, a prodrug, a deuterated product, a metabolite, a ring-opening product, or a salt of the ring-opening product thereof for use in preventing, relieving, or treating peripheral neuropathy. The peripheral neuropathy is preferably a drug-induced peripheral neuropathy, more preferably a chemotherapeutic drug-induced peripheral neuropathy.

In some embodiments, the preventing, relieving, or treating includes administering to a subject or patient a therapeutically effective amount of butylphthalide or the optical isomer, the prodrug, the deuterated product, the metabolite, the ring-opening product, or the salt of the ring-opening product thereof.

In some embodiments, the administration is selected from oral administration, injection administration, topical administration, or in vitro administration, and preferably oral administration or injection administration. The therapeutically effective amount can prevent, relieve, or treat the peripheral neuropathy in the subject or patient.

In some embodiments, the administration is oral administration. The therapeutically effective amount refers to a daily dose from about 1 mg to about 10,000 mg, preferably from about 10 mg to about 5,000 mg, or from about 20 mg to about 3,000 mg, or from about 30 mg to about 2,000 mg, or from about 50 mg to about 1,500 mg, or from about 70 mg to about 1,200mg, or from about 100 mg to about 1,000 mg, or from about 200 mg to about 900 mg, or from about 300 mg to about 800 mg, or from about 400 mg to about 700 mg, or from about 500 mg to about 600 mg, or from about 60 mg to about 800 mg, or from about 60 mg to about 600 mg, or from about 100 mg to about 800 mg, or from about 100 mg to about 600 mg, or from about 200 mg to about 600 mg, or from about 200 mg to about 800 mg, or from about 300 mg to about 600 mg, or from about 400 mg to about 600 mg, or from about 400 mg to about 800 mg, on the basis of butylphthalide.

In some embodiments, the administration is injection administration. The therapeutically effective amount refers to a daily dose from about 1 mg to about 1,000 mg, preferably from about 5 mg to about 500 mg, or from about 10 mg to about 300 mg, or from about 15 mg to about 200 mg, or from about 20 mg to about 150 mg, or from about 25 mg to about 120 mg, or from about 30 mg to about 100 mg, or from about 35 mg to about 90 mg, or from about 40 mg to about 80 mg, or from about 45 mg to about 70 mg, or from about 50 mg to about 60 mg, or from about 1 mg to about 100 mg, or from about 2 mg to about 80 mg, or from about 5 mg to about 75 mg, or from about 10 mg to about 50 mg, or from about 15 mg to about 50 mg, or from about 20 mg to about 50 mg, or from about 25 mg to about 75 mg, or from about 25 mg to about 50 mg, on the basis of butylphthalide.

In some embodiments, the butylphthalide or the optical isomer, the prodrug, the deuterated product, the metabolite, the ring-opening product, or the salt of the ring-opening product thereof is used in a single dose form or a divided dose form.

In some embodiments, the method for preventing, relieving, or treating includes: administration once per day by administering from about 60 mg to about 800 mg of the oral formulation of butylphthalide or the optical isomer, the prodrug, the deuterated product, the metabolite, the ring-opening product, or the salt of the ring-opening product thereof each time, e.g., administering about 60 mg, about 100 mg, about 200 mg, about 300 mg, about 400 mg, about 500 mg, about 600 mg, about 700 mg, or about 800 mg of the oral formulation each time, on the basis of butylphthalide; or, administration twice per day by administering from about 30 mg to about 400 mg of the oral formulation of butylphthalide or the optical isomer, the prodrug, the deuterated product, the metabolite, the ring-opening product, or the salt of the ring-opening product thereof each time, e.g., administering about 30 mg, about 100 mg, about 200 mg, about 300 mg, or about 400 mg of the oral formulation each time, on basis of butylphthalide; or, administration thrice per day by administering from about 20 mg to about 300 mg of the oral formulation of butylphthalide or the optical isomer, the prodrug, the deuterated product, the metabolite, the ring-opening product, or the salt of the ring-opening product thereof each time, e.g., administering about 20 mg, about 100 mg, about 200 mg, or about 300 mg of the oral formulation each time, on the basis of butylphthalide; or administration once per day by administering from about 1 mg to about 100 mg, preferably about 1 mg, about 2 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg, of the injection formulation of butylphthalide or the optical isomer, the prodrug, the deuterated product, the metabolite, the ring-opening product, or the salt of the ring-opening product thereof each time, on the basis of butylphthalide; or, administration twice per day by administering from about 1 mg to about 50 mg, preferably about 1 mg, about 2 mg, about 2.5 mg, about 5 mg, about 10 mg, about 15 mg, about 20 mg, about 25 mg, about 30 mg, about 35 mg, about 40 mg, about 45 mg, or about 50 mg, of the injection formulation of butylphthalide or the optical isomer, the prodrug, the deuterated product, the metabolite, the ring-opening product, or the salt of the ring-opening product thereof each time, on the basis of butylphthalide.

In some embodiments, the chemotherapeutic drug includes, but is not limited to, one or more of the followings: (1) a chemotherapeutic drug acting on a microtubule system or an anti-mitotic chemotherapeutic drug, including: a taxane drug, such as paclitaxel, docetaxel or the like; or a vinca alkaloid drug, such as vincristine, vinblastine or the like; or an epothilone drug, such as ixabepilone or the like; or a protease inhibitor drug, such as bortezomib or the like; or (2) a chemotherapeutic drug that interferes with DNA synthesis, including a platinum drug, such as cisplatin, carboplatin, oxaliplatin or the like; or (3) an immunomodulator drug, such as thalidomide, lenalidomide or the like.

In some embodiments, the butylphthalide or the optical isomer, the prodrug, the deuterated product, the metabolite, the ring-opening product, or the salt of the ring-opening product thereof may be used in combination with one or more of other drugs for treating peripheral neuropathy, wherein the other drugs for treating peripheral neuropathy are preferably duloxetine or a salt thereof, monosialotetrahexosyl ganglioside sodium.

Another object of the present disclosure further includes providing a pharmaceutical composition that comprises butylphthalide or an optical isomer, a prodrug, a deuterated product, a metabolite, a ring-opening product, or a salt of the ring-opening product thereof, and that is used for preventing, relieving, or treating peripheral neuropathy. The peripheral neuropathy is preferably a drug-induced peripheral neuropathy, more preferably a chemotherapeutic drug-induced peripheral neuropathy.

In some embodiments, the preventing, relieving, or treating includes administering to a subject or patient a therapeutically effective amount of butylphthalide or the optical isomer, the prodrug, the deuterated product, the metabolite, the ring-opening product, or the salt of the ring-opening product thereof.

In some embodiments, the administration is selected from oral administration, injection administration, topical administration, or in vitro administration, and preferably oral administration or injection administration. The therapeutically effective amount can prevent, relieve, or treat the peripheral neuropathy in the subject or patient.

In some embodiments, the administration is oral administration. The therapeutically effective amount refers to a daily dose from about 1 mg to about 10,000 mg, preferably from about 10 mg to about 5,000 mg, or from about 20 mg to about 3,000 mg, or from about 30 mg to about 2,000 mg, or from about 50 mg to about 1,500 mg, or from about 70 mg to about 1,200mg, or from about 100 mg to about 1,000 mg, or from about 200 mg to about 900 mg, or from about 300 mg to about 800 mg, or from about 400 mg to about 700 mg, or from about 500 mg to about 600 mg, or from about 60 mg to about 800 mg, or from about 60 mg to about 600 mg, or from about 100 mg to about 800 mg, or from about 100 mg to about 600 mg, or from about 200 mg to about 600 mg, or from about 200 mg to about 800 mg, or from about 300 mg to about 600 mg, or from about 400 mg to about 600 mg, or from about 400 mg to about 800 mg, on the basis of butylphthalide.

In some embodiments, the administration is injection administration. The therapeutically effective amount refers to a daily dose from about 1 mg to about 1,000 mg, preferably from about 5 mg to about 500 mg, or from about 10 mg to about 300 mg, or from about 15 mg to about 200 mg, or from about 20 mg to about 150 mg, or from about 25 mg to

about 120 mg, or from about 30 mg to about 100 mg, or from about 35 mg to about 90 mg, or from about 40 mg to about 80 mg, or from about 45 mg to about 70 mg, or from about 50 mg to about 60 mg, or from about 1 mg to about 100 mg, or from about 2 mg to about 80 mg, or from about 5 mg to about 75 mg, or from about 10 mg to about 50 mg, or from about 15 mg to about 50 mg, or from about 20 mg to about 50 mg, or from about 25 mg to about 75 mg, or from about 25 mg to about 50 mg, on the basis of butylphthalide.

In some embodiments, the butylphthalide or the optical isomer, the prodrug, the deuterated product, the metabolite, the ring-opening product, or the salt of the ring-opening product thereof is used in a single dose form or a divided dose form.

In some embodiments, the method for preventing, relieving, or treating peripheral neuropathy comprises: administration once per day by administering from about 60 mg to about 800 mg of the oral formulation of butylphthalide or the optical isomer, the prodrug, the deuterated product, the metabolite, the ring-opening product, or the salt of the ring-opening product thereof each time, e.g., administering about 60 mg, about 100 mg, about 200 mg, about 300 mg, about 400 mg, about 500 mg, about 600 mg, about 700 mg, or about 800 mg of the oral formulation each time, on the basis of butylphthalide; or, administration twice per day by administering from about 30 mg to about 400 mg of the oral formulation of butylphthalide or the optical isomer, the prodrug, the deuterated product, the metabolite, the ring-opening product, or the salt of the ring-opening product thereof each time, e.g., administering about 30 mg, about 100 mg, about 200 mg, about 300 mg, or about 400 mg of the oral formulation each time, on the basis of butylphthalide; or, administration thrice per day by administering from about 20 mg to about 300 mg of the oral formulation of butylphthalide or the optical isomer, the prodrug, the deuterated product, the metabolite, the ring-opening product, or the salt of the ring-opening product thereof each time, e.g., administering about 20 mg, about 100 mg, about 200 mg, or about 300 mg of the oral formulation each time, on the basis of butylphthalide; or administration once per day by administering from about 1 mg to about 100 mg, preferably about 1 mg, about 2 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg, of the injection formulation of butylphthalide or the optical isomer, the prodrug, the deuterated product, the metabolite, the ring-opening product, or the salt of the ring-opening product thereof each time, on the basis of butylphthalide; or, administration twice per day by administering from about 1 mg to about 50 mg, preferably about 1 mg, about 2 mg, about 2.5 mg, about 5 mg, about 10 mg, about 15 mg, about 20 mg, about 25 mg, about 30 mg, about 35 mg, about 40 mg, about 45 mg, or about 50 mg, of the injection formulation of butylphthalide or the optical isomer, the prodrug, the deuterated product, the metabolite, the ring-opening product, or the salt of the ring-opening product thereof each time, on the basis of butylphthalide.

In some embodiments, the chemotherapeutic drug includes, but is not limited to, one or more of the followings: (1) a chemotherapeutic drug acting on a microtubule system or an anti-mitotic chemotherapeutic drug, including: a taxane drug, such as paclitaxel, docetaxel or the like; or a vinca alkaloid drug, such as vincristine, vinblastine or the like; or an epothilone drug, such as ixabepilone or the like; or a protease inhibitor drug, such as bortezomib or the like; or (2) a chemotherapeutic drug that interferes with DNA synthesis, including a platinum drug, such as cisplatin, carboplatin, oxaliplatin or the like; or (3) an immunomodulator drug, such as thalidomide, lenalidomide or the like.

In some embodiments, the butylphthalide or the optical isomer, the prodrug, the deuterated product, the metabolite, the ring-opening product, or the salt of the ring-opening product thereof may be used in combination with one or more of other drugs for treating peripheral neuropathy, wherein the other drugs for treating peripheral neuropathy are preferably duloxetine or a salt thereof, and monosialotetrahexosyl ganglioside sodium.

The optical isomer of the butylphthalide according to the present disclosure is L-butylphthalide, D-butylphthalide, or a mixture of the two isomers in any ratio.

The prodrug of the butylphthalide according to the present disclosure refers to a compound that is designed according to the prodrug principle. Such compound may be metabolized into butylphthalide in vivo to realize the same or similar pharmacological effects as butylphthalide.

The deuterated product of the butylphthalide according to the present disclosure means that one or some hydrogen atoms in the structure of the butylphthalide compound is/are substituted with isotopic atom deuterium. Such deuterated product has the same or similar pharmacological effects as butylphthalide, such as deuterated derivatives of butylphthalide and its derivatives disclosed in WO2019242765A1, and particularly deuterated derivatives of butylphthalide having the following structures:

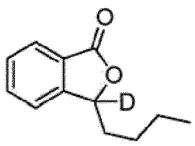 , 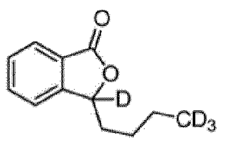 , 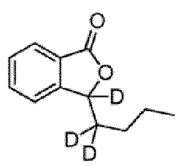 , and 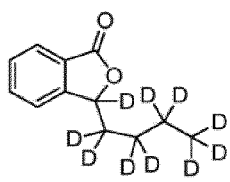 .

The metabolite of the butylphthalide according to the present disclosure refers to in vivo metabolite of butylphthalide or a derivative thereof. Such metabolite can realize the same or similar pharmacological effects as butylphthalide, such as derivatives MI and MII of butylphthalide disclosed in CN1689563A, as well as esters or salts of MI disclosed in CN102503919A or CN101289438A:

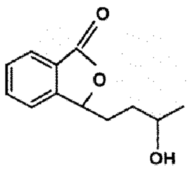

3-(3'-hydroxy)butylphthalide (Metabolite I, MI)

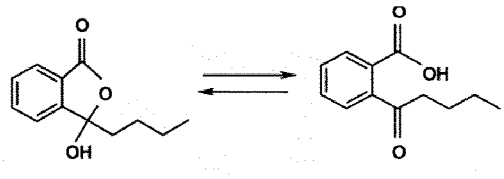

3-hydroxy-3-butylphthalide (Metabolite II, MII);

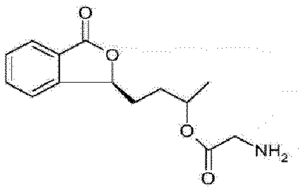
,
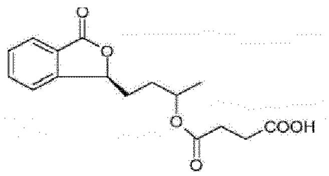
,
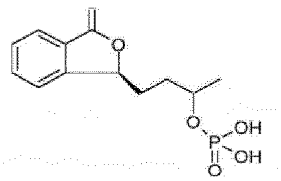
,

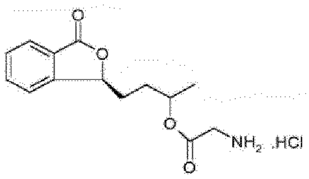
,
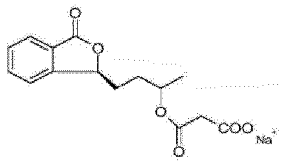
,
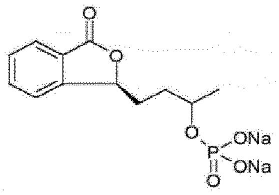
;

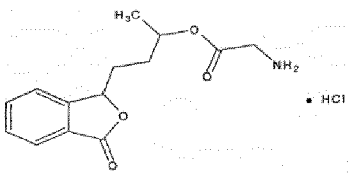
,
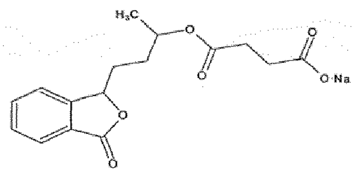
, and
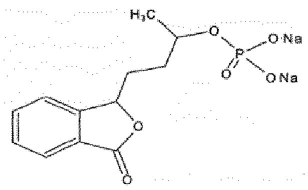
.

The ring-opening product or the salt of the ring-opening product of the butylphthalide according to the present disclosure refers to the product or salt that is obtained by ring-opening the lactone in the structure of butylphthalide. Such ring-opening product can synthesize butylphthalide in vivo by ring closure, to realize the same or similar pharmacological effects as butylphthalide, such as the ring-opening product of butylphthalide or the salt of the ring-opening product disclosed in CN1382682A, CN1523003A, and CN104974050A:

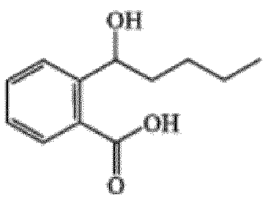

; and

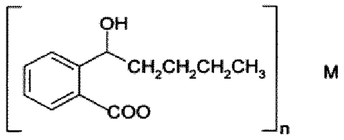

,

wherein M is a monovalent metal ion, which is a potassium ion, a sodium ion, or a lithium ion; or a divalent metal ion, which is a calcium ion, a magnesium ion, or a zinc ion, where n=1, or n=2; and M is preferably a potassium ion;

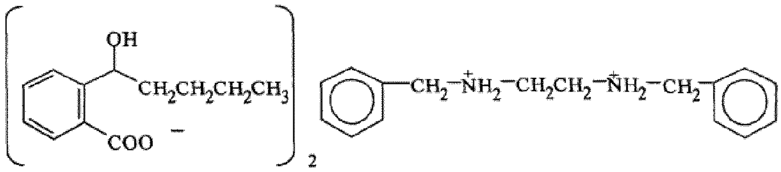

;

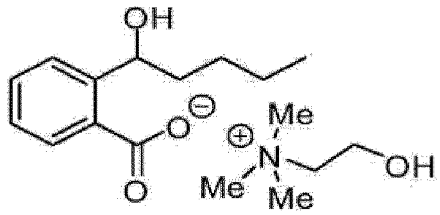

and an optical isomer thereof;

and a ring-opening derivative of glycosyl-modified butylphthalide disclosed in CN108084233A, a ring-opening derivative of butylphthalide disclosed in CN109503548A, an organic amine ester derivative of 2-(α-hydroxypentyl)benzoic acid disclosed in CN108715579A, and the like.

The dose for butylphthalide or the optical isomer, the prodrug, the deuterated product, the metabolite, the ring-opening product, or the salt of the ring-opening product thereof according to the present disclosure is based on butylphthalide.

Those skilled in the art can understand that other butylphthalide analogs or optical isomers, prodrugs, deuterated products, metabolites, ring-opening products, or salts of the ring-opening products thereof, such as 7-hydroxybutylphthalide disclosed in CN106214674A, halobutylphthalide (particularly 5-bromobutylphthalide) disclosed in CN101029037A, halogenated 2-(α-hydroxypentyl)benzoate disclosed in CN101402565A, and sodium 5-bromo-2-(α-hydroxypentyl)benzoate disclosed in CN104086399A, will also realize similar pharmacological effects as butylphthalide.

In the embodiments of the present disclosure, the word "about" before a numerical value refers to ±10%, preferably ±5%, of the numerical value, e.g., ±10%, ±9%, ±8%, ±7%, ±6%, ±5%, ±4%, ±3%, ±2%, or ±1% of the numerical value.

Technical Effects

1. The representative drug butylphthalide according to the present disclosure can significantly improve the peripheral neuropathy induced by paclitaxel or bortezomib, and can obviously reverse the symptoms of abnormal thermal pain threshold and mechanical pain threshold caused by the two chemotherapeutic drugs.

2. The representative drug butylphthalide according to the present disclosure neither will weaken the activity of bortezomib and paclitaxel in inhibiting the proliferation of tumor cells cultured in vitro, nor has significant influences on the in vivo tumor-inhibiting effect and pharmacokinetic properties of bortezomib and paclitaxel.

3. In vitro and in vivo experiments show that the drug according to the present disclosure can prevent or alleviate peripheral neuropathy caused by a chemotherapeutic drug without affecting the anti-tumor efficacy and pharmacokinetic properties of the chemotherapeutic drug.

BRIEF DESCRIPTION OF THE DRAWINGS

Fig. 1 shows a schematic diagram of a modeling method in Example 1.

Fig. 2 shows a diagram of the change in body weight of rats in each group from 0 to 16 days (D0-16) in Example 1. Compared with a model group, *P≤0.05, **P≤0.01, and ***P≤0.001.

Fig. 3 shows a diagram of the change in thermal pain threshold of rats in each group from 0 to 13 days (D0-13) in Example 1. Compared with the model group, *P≤0.05, **P≤0.01, and ***P≤0.001.

Fig. 4 shows the comparison of thermal pain thresholds of rats in each group on days 7/10/13 (D7/D10/D13) in Example 1. Compared with the model group, *P≤0.05, **P≤0.01, and ***P≤0.001.

Fig. 5 shows a time axis of modeling and administration in Example 2.

Fig. 6 shows a curve diagram of the changing values of body weight of rats in Example 2 (mean ±SD, n=12). Compared with the model group, *P≤0.05, **P≤0.01, and ***P≤0.001.

Fig. 7 shows the change of thermal pain threshold of rats on days -1/7/14 in Example 2 (mean ±SD, n=12). Compared with the model group, *P≤0.05, **P≤0.01, and ***P≤0.001.

Fig. 8 shows the change of mechanical pain threshold of rats on days 0/6/13 in Example 2 (mean ±SD, n=12). Compared with the model group, *P≤0.05, **P≤0.01, and ***P≤0.001.

Fig. 9 shows the change of body weight of rats in each group in Example 3. Compared with a solvent group, *P≤0.05, **P≤0.01, and ***P≤0.001; and compared with an ab-PTX group, ##P≤0.01.

Fig. 10 shows the change of body weight of rats in each group in Example 4. According to statistical analysis, compared with the solvent group, *P≤0.05, **P≤0.01, and ***P≤0.001.

Fig. 11 shows a time axis of modeling and administration in Example 6.

Fig. 12 shows the change of body weight of rats in each group on D0-D20 in Example 6. Compared with the model group, *P≤0.05, **P≤0.01, and ***P≤0.001.

Fig. 13 shows the change of thermal pain threshold of rats in Example 6. Compared with the model group, *P≤0.05, **P≤0.01, and ***P≤0.001.

Fig. 14 shows the change of mechanical pain threshold of rats in Example 6. Compared with the model group, *P≤0.05, **P≤0.01, and ***P≤0.001.

Fig. 15 shows a curve diagram of the changing values of body weight of mice in each group in Example 8 (mean ±SD, n=6).

Fig. 16 shows a curve diagram of the change of xenograft tumor volume of tumor-bearing mice in Example 8 (mean ±SD, n=6; and compared with the solvent group, *P≤0.05, **P≤0.01, and ***P≤0.001).

Note: mpk in the figures is equivalent to mg/kg. DETAILED DESCRIPTION OF EMBODIMENTS

The examples listed below are provided to better illustrate the embodiments of the present disclosure, but the present disclosure is not limited to the listed examples. Those skilled in the art can make non-essential improvements and adjustments to the embodiments according to the above content of the disclosure, and the improved and adjusted embodiments still belong to the scope of the present disclosure.

Example 1: Study on in vivo efficacy of butylphthalide (intraperitoneal injection) on paclitaxel (albumin bound) chemotherapy-induced peripheral neuropathy in rats

1. Experimental animals

59 male SD (Sprague Dawley) rats, 180-200 g.

2. Drugs

Lyophilized powder of paclitaxel (albumin bound, ab-PTX), 100 mg/bottle, made by CSPC Ouyi Pharmaceutical Co., Ltd, batch number: B041909121, dissolved in normal saline immediately prior to use.

Butylphthalide (NBP) injection, 25 mg/5 mL, made by CSPC NBP Pharmaceutical Co., Ltd, batch number: Q27190401, diluted in normal saline to a target concentration immediately prior to use.

3. Experimental process

(1) Model preparation and administration method

Male SD rats, ab-PTX 5 mg/kg, administration volume: 5 mL/kg, intraperitoneally injected (i.p.) on days 0, 2, 4, 6, 8, and 11 for modeling. NBP was administered at three doses (1.5, 5, and 15 mg/kg bid), starting on 1 day prior to the ab-PTX modeling, and during the ab-PTX administration and modeling, the first dose of NBP was administered by intraperitoneal injection 1 h prior to the ab-PTX administration. The second dose of NBP was administered with an interval of 4 h from the first dose, with an administration volume of 5 mL/kg, and was administered every day for 17 consecutive days. The normal group was administered with 0.9% normal saline (0.9% INJ NS) at the same frequency. The modeling method is shown in Fig. 1. The animal grouping, the administration method, and the doses are detailed in Table 3.

Table 3 Animal Grouping and Dose List in Example 1

| Group | Number of animals | Drug | Dose (mg/kg) | Administration route |
|---|---|---|---|---|
| Normal group | 11 | 0.9% INJ NS | --- | i.p. |
| Model group | 12 | 0.9% INJ NS/ab-PTX | -/5 | i.p./i.p. |
| NBP 1.5 mg/kg/bid | 12 | NBP/ab-PTX | 1.5/5 | i.p./i.p. |
| NBP 5 mg/kg/bid | 12 | NBP/ab-PTX | 5/5 | i.p./i.p. |
| NBP 15 mg/kg/bid | 12 | NBP/ab-PTX | 15/5 | i.p./i.p. |
| Note: (1) bid: twice per day; and (2) the three doses of NBP were converted into adult daily doses (based on 60 kg, oral administration) of 60 mg/d, 200 mg/d, and 600 mg/d, respectively. | | | | |

(2) Grouping

Based on the thermal pain threshold baselines and the body weights of the rats, the rats with the baseline values ranging from 3 to 6 s were selected, and were equally grouped in accordance with the above Table 3.

(3) Observation indexes

① Body weight: all animals were weighed once prior to the experiment, and were weighed at set time every day after the commencement of administration, but only the body weight data on days -1, 0, 2, 4, 6, 8, 10, 12, 14, and 16 were statistically recorded.

② Thermal pain threshold: IITC Life Science Electronic Von Frey Anesthesio meter was used and the intensity of the light source was adjusted to obtain a baseline of thermal pain threshold of about 3-6 s. In this case, the light intensity was 58%, and the cutoff value was set as 10 s. The rats were placed in a plastic compartment on a glass plate, to make the planta centers of the rats be exposed to the light source. The time of reflexive withdrawal, i.e., the thermal pain threshold, of the rat was recorded, and measured once for each of the left foot and the right foot of each rat, and the measurement was repeated three times with each interval therebetween ≥15 min, to compute a mean value of the 6 thermal pain thresholds of each rat.

4. Experimental results

(1) Body weight

On days 4-16 (D4-16) of modeling, compared with the normal group, the body weights of the rats in the model group were significantly decreased (*P*<0.01); and compared with the model group, the body weights of the rats in the three dose groups of NBP (1.5, 5, and 15 mg/kg bid) were not significantly changed, as detailed in Fig. 2.

(2) Thermal pain threshold

Measurements were performed on days 7, 10, 13, and 17. On days 7-13 (D7-13), compared with the normal group, the thermal pain threshold of the model group was significantly increased (*P*<0.01); and compared with the model group, the increase of the thermal pain threshold of the model rats in the groups of NBP (1.5, 5, and 15 mg/kg bid) can be significantly decreased (*P*<0.05 or *P*<0.01), as detailed in Fig. 3 and Fig. 4.

On day 17 (D17), compared with the normal group, there was no statistical difference in the thermal pain threshold of the rats in the model group (D17 data was not shown), therefore the thermal pain threshold of the NBP therapy group was not detected, and the experiment was terminated.

5. Conclusions

Butylphthalide can effectively alleviate paclitaxel (albumin bound)-induced abnormal thermal pain symptoms of peripheral neuropathy in rats.

Example 2: Study on in vivo efficacy of butylphthalide (intragastric administration) on paclitaxel (albumin bound) chemotherapy-induced peripheral neuropathy in rats

1. Experimental animals

72 male SD (Sprague Dawley) rats, 160-180 g.

2. Drugs

Lyophilized powder of paclitaxel (albumin bound, ab-PTX), 100 mg/bottle, made by CSPC Ouyi Pharmaceutical Co., Ltd, batch number: B041909121, dissolved in normal saline immediately prior to use.

Butylphthalide (oral administration grade), 10 kg/bottle, made by CSPC NBP Pharmaceutical Co., Ltd, batch number: 518180803, diluted in a vegetable oil to a target concentration immediately prior to use.

Duloxetine hydrochloride, batch number: QRQYD-JN, TCI (Shanghai) Development Co., Ltd, prepared with normal saline containing 2% DMSO immediately prior to use.

3. Experimental process

3.1 Model preparation and administration method

In this experiment, male SD rats were administered with 5 mg/kg of ab-PTX with an administration volume of 5 mL/kg, and intraperitoneally injected (i.p.) on days 0, 2, 4, 6, 9, and 13 (the first dose of ab-PTX was administered on day 0 of the experiment) for modeling.

Three doses of NBP (3, 10, and 30 mg/kg bid) were used, and the positive control drug (duloxetine hydrochloride) was 15 mg/kg qd. Both NBP and duloxetine hydrochloride were administered 1 day prior to the ab-PTX modeling. During the ab-PTX administration and modeling, both NBP (first dose) and duloxetine hydrochloride were administered 1 h prior to intraperitoneal injection of ab-PTX. The second dose of NBP was administered with an interval of ≥4 h from the first dose, with an administration volume of 5 mL/kg, and was administered by intragastric administration for 14 consecutive days.

The model group was given vegetable oil at the same frequency and with the same administration method as NBP.

The normal group was given normal saline at the same frequency and with the same administration method as ab-PTX, and was given vegetable oil at the same frequency and with the same administration method as NBP.

The modeling method and the administration time are shown in Fig. 5. The animal grouping, the administration method, and the doses are detailed in Table 4.

Table 4 Animal Grouping and Dose List

| Group | Number of animals | Drug | Dose (mg/kg) | Administration route |
|---|---|---|---|---|
| Normal group | 12 | Vegetable oil/normal saline | --- | p.o./i.p. |
| Model group | 12 | Vegetable oil/ab-PTX | 5 | p.o./i.p. |
| NBP 3 mg/kg bid | 12 | NBP/ab-PTX | 3/5 | p.o./i.p. |
| NBP 10 mg/kg bid | 12 | NBP/ab-PTX | 10/5 | p.o./i.p. |
| NBP 30 mg/kg bid | 12 | NBP/ab-PTX | 30/5 | p.o./i.p. |
| Duloxetine hydrochloride 15 mg/kg qd | 12 | Duloxetine hydrochloride/ab-PTX | 15/5 | p.o./i.p. |
| Note: (1) p.o.: intragastric administration; (2) i.p.: intraperitoneal injection; (3) the three doses of NBP were converted into adult daily doses (based on 60 kg, oral administration) of 60 mg/d, 200 mg/d, and 600 mg/d, respectively. | | | | |

3.2 Grouping

Based on the thermal pain threshold baseline values and the body weights of the rats, the rats with the baseline values ranging from 3 to 6 s were selected, and were equally grouped in accordance with Table 4 in 3.1.

3.3 Observation indexes

(1) Body weight: all animals were weighed once prior to the experiment, and were weighed at set time every day after the commencement of administration, but only the body weight data on days -1, 0, 2, 4, 6, 8, 10, 12, and 14 were statistically recorded.

(2) Thermal pain threshold: measurements were performed on days -1, 7, and 14.
Method: IITC Life Science Electronic Von Frey Anesthesio meter was used and the intensity of the light source was adjusted to obtain a baseline of thermal pain threshold of about 3-6 s. In this case, the light intensity was 58%, and the cutoff value was set as 10 s. The rats were placed in a plastic compartment on a glass plate, to make the planta centers of the rats be exposed to the light source. The time of reflexive withdrawal, i.e., the thermal pain threshold, of the rat was recorded, and was measured once for each of the left foot and the right foot of each rat, and the measurement was repeated three times with each interval therebetween ≥15 min, to compute a mean value of the 6 thermal pain thresholds of each rat.

(3) Mechanical stimulus pain threshold: measurements were performed on days 0, 6, and 13.
Method: the rats were placed in a plastic compartment on a wire mesh, left to stand still for 15 min, stimulated using a stimulation probe with a diameter of 0.4 mm of the IITC Life Science Electronic Von Frey Anesthesio meter at the planta centers of the rats by gradually increasing the force until reflexive withdrawal of the rats. The maximum value displayed by the instrument at this time, i.e., the mechanical pain threshold, was recorded. In each experiment, the left and right feet of each rat were measured three times with each interval therebetween ≥5 min, to compute a mean value of the 6 mechanical pain thresholds of each rat.

4. Results

4.1 Influence on body weight

On days 4-14 of the experiment, compared with the normal group, the body weights of the rats in the model group were obviously decreased (*P*<0.05); and throughout the administration period, compared with the model group, the body weights of the rats in the three dose groups of NBP (3, 10, and 30 mg/kg bid) were not obviously changed, and the positive drug duloxetine hydrochloride can significantly increase the body weights of the model rats (*P*<0.05), as detailed in Fig. 6.

4.2 Influence on thermal pain threshold

On day 7 of the experiment, compared with the normal group, the thermal pain threshold of the rats in the model group was significantly increased ($P$<0.01); and compared with the model group, the thermal pain threshold of the rats in the group of NBP (30 mg/kg bid) was significantly decreased ($P$<0.05).

On day 14 of the experiment, compared with the normal group, the thermal pain threshold of the rats in the model group was significantly increased ($P$<0.001); and compared with the model group, the thermal pain thresholds of the rats in the three dose groups of NBP (3, 10, and 30 mg/kg bid) and the group of positive drug duloxetine hydrochloride (15 mg/kg qd) were significantly decreased ($P$<0.05), as detailed in Fig. 7.

4.3 Influence on mechanical pain threshold

On day 6 of the experiment, compared with the normal group, the mechanical pain threshold of the rats in the model group was significantly increased ($P$<0.05); and compared with the model group, the mechanical pain threshold of the rats in the group of NBP (30 mg/kg bid) was significantly decreased ($P$<0.05).

On day 13 of the experiment, compared with the normal group, the mechanical pain threshold of the rats in the model group was significantly increased ($P$<0.001); and compared with the model group, the mechanical pain thresholds of the rats in the dose groups of NBP (10 and 30 mg/kg bid) and the group of positive drug duloxetine hydrochloride (15 mg/kg qd) were significantly decreased ($P$<0.05), and the mechanical pain threshold of the rats in the dose group of NBP (3 mg/kg bid) had a decreasing trend, but did not have statistical difference, as detailed in Fig. 8.

6. Conclusions

Under the conditions of this experiment, NBP can dose-dependently effectively relieve the symptoms of peripheral neuropathy in an ab-PTX-induced PIPN (Paclitaxel-Induced Peripheral Neuropathy) model of rats.

Example 3: Influence of NBP on antitumor efficacy and PK of ab-PTX against B16/F10 xenograft tumor

1. Experimental system

1.1 Experimental animals

40 female C57BL/6N mice, 15-17 g.

1.2 Cell strains

B16/F10 cells (mice melanoma cells): purchased from Shanghai Genechem Co., Ltd.

2. Drugs

Lyophilized powder of paclitaxel (albumin bound), 100 mg/bottle, made by CSPC Ouyi Pharmaceutical Co., Ltd, batch number: B041909121, dissolved in normal saline immediately prior to use.

Butylphthalide, 25 mg/5 mL/bottle, made by CSPC NBP Pharmaceutical Co., Ltd, batch number: Q27190401, diluted in normal saline to a target concentration immediately prior to use.

3. Experimental process

3.1 Model preparation

After in vitro resuscitation and passage of B16/F10 cells to a sufficient amount, the cells were counted by a microscope, and diluted with a serum-free medium to adjust the cell count to about $1\times10^7$ cells/mL. The cell suspension was kept in an ice-water bath.

The B16/F10 cell suspension was extracted by a sterile syringe, and inoculated into the subcutaneous tissue of the forelimb axilla of the C57BL/6N mice, with the inoculation volume of 0.1 mL/mouse containing about $1.0\times10^6$ tumor cells, to prepare B16/F10 xenograft tumor model of the C57BL/6N mice.

3.2 Administration method

Therapy group:
Monotherapy group of ab-PTX: intraperitoneal injection of ab-PTX at 25 mg/kg, biw (on days 1, 4, 8, 11, and 15). Administration volume: 10 mL/kg.

NBP/ab-PTX group: ab-PTX was administered by intraperitoneal injection at 25 mg/kg, biw (on days 1, 4, 8, 11, and 15); NBP was administered by intraperitoneal injection at 3 mg/kg, 10 mg/kg, or 30 mg/kg twice per day, bid (administration was commenced on day 0); and during the ab-PTX modeling, the first dose of NBP was administered by intraperitoneal injection 1 h prior to the ab-PTX administration, and the second dose of NBP was administered with an interval of more than 4 h from the first dose. The administration was performed for 15 consecutive days. The administration volume was 10 mL/kg.

Solvent group: the solvent (normal saline) was intraperitoneally injected at the same frequency and with the same administration volume as ab-PTX and NBP.

Note: the three doses of NBP were converted into adult daily doses (based on 60 kg, oral administration) of 60 mg/d, 200 mg/d, and 600 mg/d, respectively.

3.3 Observation indexes and evaluation indexes

3.3.1 General observation indexes

(1) General state observation: all animals were observed once per day during the experimental period, and the abnormality and behavioral changes of their body parts were recorded.

(2) Body weight: all animals were weighed once prior to the experiment, and animals having suitable body weights were selected for the experiment. Animals were weighed at set time once per day after the commencement of administration.

(3) Death and near death: the death time of dead animal was recorded, and dying animal should be observed more frequently to determine the death time.

3.3.2 Tumor weight

At the end of the experiment, after the animals were euthanized by $CO_2$ asphyxiation, the tumors were excised and weighed.

Tumor weight inhibition rate% = (1−tumor weight in therapy group/tumor weight in solvent group) × 100%

3.3.3 PK blood sampling and detection

Before the administration of the last dose of ab-PTX (0 h), and 5 min, 0.5 h, 1 h, 4 h, 8 h, 12 h, 24 h after the administration, blood was sampled (as detailed in Table 5), anticoagulated with heparin, and centrifuged to separate plasma, which was stored at - 80 °C for later use. The total amount of paclitaxel in the plasma was determined by protein precipitation-LC/MS/MS.

Table 5 List of Blood Sampling Time of Mice in Each Group

| Group | Animal No. | Blood sampling time |
|---|---|---|
| Solvent group | 1-8 | 0 h |
| Therapy groups | 1-4 | 0 h, 0.5 h, 8 h, 12 h |
| Therapy groups | 5-8 | 5 min, 1 h, 8 h, 24 h |

4. Results

4.1 Influence on body weight

Compared with the solvent group, the body weight gain rate of mice in each therapy group was significantly decreased (*P*<0.01). Compared with the ab-PTX group, the body weight gain rate of the dose group of NBP/ab-PTX (10/25 mg/kg) was significantly decreased (*P*<0.01). Based on analysis of the tumor weight data, the decrease in the body weight gain rate of each therapy group should be caused by inhibition of the drug on the tumor weight, as detailed in Fig. 9.

4.2 Influence on tumor weight

Compared with the solvent group, the tumor growth of each therapy group can be significantly inhibited (*P*<0.001); and compared with the ab-PTX group, the combined therapy of different doses of NBP and ab-PTX did not have statistical difference in tumor inhibition (*P*>0.05). The tumor weight of each group is detailed in Table 6.

Table 6 Influence on Tumor Weights of Mice (mean ±SD, n=8)

| Group | Number of animals (surviving/total) | Tumor weight (g) | Inhibition rate (%) |
|---|---|---|---|
| Solvent group | 8 / 8 | 5.2178±1.5146 | --- |
| NBP/ab-PTX 3/25 mg/kg | 8 / 8 | 1.9635±0.7882*** | 62.4 |
| NBP/ab-PTX 10/25 mg/kg | 8 / 8 | 1.7374±0.5333*** | 66.7 |
| NBP/ab-PTX 30/25 mg/kg | 8 / 8 | 1.8226±0.9578*** | 65.1 |
| ab-PTX 25 mg/kg | 8 / 8 | 2.5024±0.8133*** | 52.0 |
| Note: Compared with the solvent group: ****P*<0.001. | | | |

4.3 Influence on PK

The total amount of paclitaxel in the plasma was determined by protein precipitation-LC/MS/MS. The results showed that there was no significant statistical difference in $C_{max}$, $AUC_{0-t}$, and other indexes in each therapy group, indicating that NBP did not affect the PK behavior of ab-PTX in mice, as detailed in Fig. 7.

Table 7 Influence on Pharmacokinetic Parameters

| Group | $C_{max}$ (ng/ml) | $AUC_{0-t}$ (h*ng/ml) | $AUC_{0-\infty}$ (h*ng/ml) | $T_{max}$ (h) | $t_{1/2}$ (h) |
|---|---|---|---|---|---|
| NBP/ab-PTX 3/25 mg/kg | 3157 | 8012 | 8065 | 0.5 | 1.55 |
| NBP/ab-PTX 10/25 mg/kg | 2315 | 7178 | 7226 | 0.5 | 1.72 |
| NBP/ab-PTX 30/25 mg/kg | 2210 | 6973 | 7130 | 1.0 | 2.31 |
| ab-PTX 25 mg/kg | 2443 | 8126 | 8178 | 0.5 | 1.55 |

5. Conclusions

In this experiment, NBP did not have obvious influence on the antitumor efficacy of ab-PTX and the PK behavior of ab-PTX.

Example 4 Influence of NBP on antitumor efficacy and PK of ab-PTX against JIMT-1 xenograft tumor

1. Experimental system

1.1 Experimental animals

48 female Nu/Nu mice, 15-17 g.

1.2 Cell strains

JIMT-1 cells (human breast cancer cells): purchased from Nanjing Cobioer Biosciences Co., Ltd.

2. Experimental objective

JIMT-1 cell xenograft model of the Nu/Nu mice was used in this experiment to validate the influence of NBP on the antitumor efficacy and PK behavior of ab-PTX.

3. Drugs

Lyophilized powder of paclitaxel (albumin bound), 100 mg/bottle, batch number: B042007410, made by CSPC Ouyi Pharmaceutical Co., Ltd, dissolved in normal saline immediately prior to use.

Butylphthalide (oral administration grade), 10 kg/bottle, batch number: 518180803, made by CSPC NBP Pharmaceutical Co., Ltd, diluted with vegetable oil to a target concentration immediately prior to use.

4. Experimental process

4.1 Model preparation

After in vitro resuscitation and passage of JIMT-1 cells to a sufficient amount, the cells were counted by a microscope, and diluted with a serum-free medium to adjust the cell count to about $1\times10^8$ cells/mL. The cell suspension was kept in an ice-water bath.

The JIMT-1 cell suspension was extracted by a sterile syringe and inoculated into the subcutaneous tissue of the forelimb axilla of the Nu/Nu mice, with the inoculation volume of 0.1 mL/mouse containing about $1.0\times10^7$ tumor cells, to prepare JIMT-1 xenograft tumor model of the Nu/Nu mice.

4.2 Administration method

Therapy groups:
Monotherapy group of ab-PTX: intravenous injection of ab-PTX at 15 mg/kg once every week, qw (on days 0, 7, 14, 21, and 28). Administration period: 28 days. Administration volume: 10 mL/kg.

Monotherapy group of NBP: NBP was administered by transoral gavage at 60 mg/kg, bid (administration was commenced on day 0), the dosing interval should be longer than 4 h; and the administration period was 28 days. The administration volume was 10 mL/kg.

NBP/ab-PTX group: intravenous injection of ab-PTX at 15 mg/kg, qw (on days 0, 7, 14, 21, and 28). NBP was administered by transoral gavage at 6 mg/kg, 20 mg/kg, or 60 mg/kg twice per day, bid (administration was commenced on day 0), the dosing interval should be longer than 4 h; the first dose of NBP was administered 1 h prior to the ab-PTX administration. The administration period was 28 days. The administration volume was 10 mL/kg.

The solvent group was given normal saline at the same frequency and with the same method as ab-PTX, and was given vegetable oil at the same frequency and with the same method as NBP.

Note: The administration doses of NBP were converted into adult daily doses (based on 60 kg, oral administration) of 60 mg/d, 200 mg/d, and 600 mg/d, respectively.

4.3 Observation indexes and evaluation indexes

4.3.1 General observation indexes

(1) General state observation: all animals were observed once per day during the experimental period, and the abnormality and behavioral changes of their body parts were recorded.

(2) Body weight: all animals were weighed once prior to the experiment, and animals having suitable body weights were selected for the experiment. Animals were weighed at set time once per day after the commencement of administration.

(3) Death and near death: the death time of dead animals was recorded, and dying animals should be observed

more frequently to determine the death time.

4.3.2 Tumor weight

At the end of the experiment, after the animals were euthanized by $CO_2$ asphyxiation, the tumors were excised and weighed.

$$\text{Tumor weight inhibition rate\%} = (1-\text{tumor weight in therapy group/tumor weight in solvent group}) \times 100\%$$

4.3.3 PK blood sampling and detection

Before administration of the last dose of ab-PTX (0 h), and 5 min, 15 min, 0.5 h, 1 h, 4 h, 8 h, 24 h after the administration, blood was sampled (as detailed in Table 8), anticoagulated with heparin, and centrifuged to separate plasma, which was stored at - 80 °C for later use. The total amount of paclitaxel in the plasma was determined by protein precipitation-LC/MS/MS.

Table 8 List of Blood Sampling Time of Mice in Each Group

| Group | Animal No. | Blood sampling time |
|---|---|---|
| Solvent group | 1-8 | 0 h |
| Therapy | 1-8 | 0 h, 5 min, 15 min, 0.5 h, 1 h, 4 h, 8 h, 24 h |
| groups | | |

5. Results

5.1 Influence on body weight

As can be seen from the body weight data, there was no significant difference in the body weights of the mice in each group, indicating that neither ab-PTX nor NBP had significant influence on the body weights of the mice, as detailed in Fig. 10.

5.2 Influence on tumor weight

The tumor weight results showed that, compared with the solvent group, the tumor growth of the ab-PTX therapy group can be significantly inhibited (*P*<0.001), and the tumor growth of the mice in the NBP dose group of 60 mg/kg was not obviously affected; compared with the ab-PTX group of 15 mg/kg, the combined therapy of different doses of NBP and ab-PTX did not have statistical difference in tumor inhibition (*P*>0.05), as detailed in Table 9.

Table 9 Influence on Tumor Weights of Mice (mean ±SD, n=8)

| Group | Number of animals (surviving/total) | Tumor weight (g) | Inhibition rate (%) |
|---|---|---|---|
| Solvent group | 8/8 | 0.9969±0.2426 | --- |
| ab-PTX 15 mg/kg | 8/8 | 0.2789±0.0858*** | 72.0 |
| NBP/ab-PTX 6/15 mg/kg | 8/8 | 0.2466±0.1651*** | 75.3 |
| NBP/ab-PTX 20/15 mg/kg | 8/8 | 0.2807±0.1309*** | 71.8 |
| NBP/ab-PTX 60/15 mg/kg | 8/8 | 0.3140±0.1724*** | 68.5 |
| NBP 60 mg/kg | 8/8 | 0.9570±0.2529 | 4.0 |
| Note: Compared with the solvent group: ****P*<0.001. | | | |

5.4 Influence on PK

The results showed that the main pharmacokinetic parameters of paclitaxel in the plasma of the monotherapy group of ab-PTX and the combined therapy group of NBP and ab-PTX were substantially consistent without significant difference, indicating that combined therapy of butylphthalide and ab-PTX did not affect the pharmacokinetic behavior of paclitaxel in the plasma, as detailed in Table 10.

Table 10 Main Pharmacokinetic Parameters of Total Amount of Paclitaxel in Therapy Groups

| Parameters (unit) | Mean ±SD (n=8) | | | |
|---|---|---|---|---|
| | ab-PTX | NBP/ab-PTX 6/15 mg/kg | NBP/ab-PTX 20/15 mg/kg | NBP/ab-PTX 60/15 mg/kg |
| $C_{max}$ (ng/mL) | 2981±718 | 2510±290 | 2208±382 | 3083±498 |
| $AUC_{0-t}$ (h*ng/mL) | 2526±963 | 2565±371 | 2467±633 | 2732±376 |
| $AUC_{0-\infty}$ (h*ng/mL) | 2575±992 | 2600±369 | 2491±659 | 2762±383 |
| $t_{1/2}$ (h) | 1.28±0.28 | 1.16±0.14 | 1.08±0.13 | 1.09±0.21 |
| Vss (L/kg) | 7.39±1.64 | 6.64±0.63 | 6.91±1.73 | 5.70±0.94 |
| Cl(L/h/kg) | 6.61±2.40 | 5.87±0.81 | 6.43±1.84 | 5.53±0.82 |
| $MRT_{0-t}$ (h) | 1.05±0.24 | 1.04±0.13 | 1.01±0.14 | 0.956±0.140 |

6. Conclusions

In this experiment, NBP did not have obvious influence on the antitumor efficacy of ab-PTX and the PK behavior of ab-PTX.

Example 5 Influence of NBP on in vitro efficacy of ab-PTX

1. Cell and culture conditions

Table 11 Cell and Essential Culture Medium

| Cell strains | Source | Histological source | Culture medium |
|---|---|---|---|
| MDA-MB-231 | Cell Culture Center, Peking Union Medical College | Human breast cancer cells | RPMI Medium 1640 containing 10% FBS |
| JIMT-1 | Nanjing Cobioer Biosciences Co., Ltd | Human breast cancer cells | DMEM containing 10% FBS |
| SK-OV-3 | Shanghai Institutes for Biological Sciences, Chinese Academy of Sciences | Human ovarian cancer cells | McCoy's 5A containing 10% FBS |
| HT29 | Shanghai Institutes for Biological Sciences, Chinese Academy of Sciences | Human colon cancer cells | McCoy's 5A containing 10% FBS |
| A549 | ATCC | Human lung cancer cells | RPMI Medium 1640 containing 10% FBS |
| A375 | Cell Culture Center, Peking Union Medical College | Human melanoma cells | DMEM containing 10% FBS |

The above cell culture conditions were 37 °C and 5% $CO_2$.

2. Experimental objective

To investigate whether NBP affects the function of ab-PTX in the in vitro inhibition of tumor cell proliferation.

3. Drugs

Lyophilized powder of paclitaxel (albumin bound), 100 mg/bottle, batch number: B041909121, made by CSPC Ouyi Pharmaceutical Co., Ltd.
Butylphthalide (oral administration), 10 kg/bottle, batch number: 518180803, made by CSPC NBP Pharmaceutical Co., Ltd.

4. Experimental design

This experiment includes a monotherapy group of ab-PTX, a combined therapy group (ab-PTX/NBP), and an NBP group. Different drug concentration gradients were set for each group.
Monotherapy group of ab-PTX: ① MDA-MB-231, JIMT-1, SK-OV-3, and A549 cell strains: ab-PTX was 3× serially diluted from an initial concentration of 200 nM to 8 concentrations: 200, 66.67, 22.22, 7.41, 2.47, 0.82, 0.27, and 0.09 nM, respectively. ② A375 and HT29 cell strains: in the first experiment, ab-PTX was 3× serially diluted from an initial concentration of 100 nM to 8 concentrations: 100, 33.33, 11.11, 3.70, 1.24, 0.41, 0.14, and 0.05 nM, respectively; and in the repeated experiment, ab-PTX was 2× serially diluted from an initial concentration of 100 nM to 8 concentrations: 100, 50, 25, 12.5, 6.25, 3.13, 1.56, and 0.78 nM, respectively.
ab-PTX/NBP group: the final concentration of NBP was 30 μM, and the concentration gradient of ab-PTX was the same as that of the monotherapy group.
Monotherapy group of NBP: NBP was 2× serially diluted from an initial concentration of 480 μM to 8 concentrations: 480, 240, 120, 60, 30, 15, 7.5, and 3.75 μM, respectively.
The action time of the drug was 72 h.

5. Experimental process

A certain number of conventionally cultured cells in logarithmic growth phase were inoculated in a 96-well plate with 100 μL per well. 24 h after adherence,100 μL of the culture solution containing different concentration gradients of ab-PTX was added to each well of the monotherapy group of ab-PTX; 100 μL of the culture solution containing different concentration gradients of ab-PTX and 30 μM NBP was added to each well of the combined therapy group of ab-PTX/NBP; 100 μL of the culture solution containing different concentration gradients of NBP was added to each well of the monotherapy group of NBP; 3 repeated wells were provided for each concentration of each drug, and the blank wells (only culture medium was present) and the normal wells (drug concentration was 0) were provided. The drugs functioned for 72 h, then MTT working solution (5 mg/mL) was added at 20 μL per well; after functioning at 37 °C for 4 h, the supernatant was removed, 150 μL of DMSO (analytically pure) was added; the mixture was fully mixed using a microplate oscillator, the plate was wiped clean, and the optical density (OD) at 550 nm was measured using a microplate reader.
The cell growth inhibition rate was computed using the following equation:

$$\text{Inhibition rate (\%)} = (\text{OD value}_{\text{normal well}} - \text{OD value}_{\text{administration well}}) / (\text{OD value}_{\text{normal well}} - \text{OD value}_{\text{blank well}}) \times 100\%$$

Based on the inhibition rate of each concentration, the median inhibitory concentration $IC_{50}$ of the drug was computed using SPSS19.0.
The experiment was repeated three times, and the $IC_{50}$ value was expressed as the mean ±SD of the results of the 3 experiments.

6. Results

6.1 Influence of NBP on $IC_{50}$ value of ab-PTX

The results of the monotherapy group of ab-PTX showed that ab-PTX had obvious inhibitory effect on the in vitro proliferation of human breast cancer cells (MDA-MB-231, JIMT-1), human ovarian cancer cells (SK-OV-3), human colon cancer cells (HT29), human lung cancer cells (A549), and human melanoma (A375), and the mean $IC_{50}$ value was in the range of 5-50 nM. NBP at a particular concentration (30 μM) was added for the combined therapy. Compared with the monotherapy of ab-PTX, the mean $IC_{50}$ of ab-PTX of cells in the combined therapy group was not obviously changed, and remained in the range of 5-50 nM. The result showed that NBP did not significantly affect the function of ab-PTX in the in vitro inhibition of tumor cell proliferation.
$IC_{50}$ values of the monotherapy of ab-PTX and the combined therapy of ab-PTX and NBP in inhibiting the in

vitro proliferation of 6 tumor cell strains are shown in Table 12.

Table 12 $IC_{50}$ Values (nM) of Monotherapy of ab-PTX and Combined Therapy of ab-PTX and NBP in Inhibiting the in vitro Proliferation of 6 Tumor Cell Strains

| Cell type | $IC_{50}$ (nM) | |
|---|---|---|
| | Monotherapy of ab-PTX | Combined therapy of ab-PTX/NBP |
| MDA-MB-231 | 37.11±11.78 | 47.60±9.05 |
| JIMT-1 | 5.61±2.40 | 7.24±0.96 |
| SK-OV-3 | 20.82±8.20 | 27.66±15.05 |
| HT29 | 5.03±1.39 | 5.95±1.83 |
| A549 | 31.20±16.72 | 46.23±21.37 |
| A375 | 12.18±7.49 | 17.89±7.51 |

6.2 Influence of NBP on the in vitro proliferation of tumor cells

The NBP alone did not have obvious inhibitory effect on the in vitro proliferation of human breast cancer cells (MDA-MB-231, JIMT-1), human ovarian cancer cells (SK-OV-3), human colon cancer cells (HT29), human lung cancer cells (A549), and human melanoma (A375). The inhibition rate of 30 μM NBP on the proliferation of 6 cell strains is shown in Table 13.

Table 13 Inhibition Rate (%) of 30 μM NBP Alone on 6 Cell Strains

| Cell type | Inhibition rate (%, 30 μM NBP) |
|---|---|
| MDA-MB-231 | 3.43±3.95 |
| JIMT-1 | 7.39±13.23 |
| SK-OV-3 | 12.37±7.41 |
| HT29 | 10.07±7.94 |
| A549 | 5.12±7.36 |
| A375 | 10.92±0.57 |

7. Conclusions

NBP does not affect the inhibitory effect of ab-PTX on the in vitro proliferation of human breast cancer cells (MDA-MB-231, JIMT-1), human ovarian cancer cells (SK-OV-3), human colon cancer cells (HT29), human lung cancer cells (A549), and human melanoma cells (A375); and the NBP alone did not have obvious inhibitory effect on the in vitro proliferation of human breast cancer cells (MDA-MB-231, JIMT-1), human ovarian cancer cells (SK-OV-3), human colon cancer cells (HT29), human lung cancer cells (A549), and human melanoma (A375).

Example 6: Study on in vivo efficacy of butylphthalide on bortezomib chemotherapy-induced peripheral neuropathy

1. Experimental animals

72 male SD (Sprague Dawley) rats, 180-200 g.

2. Drugs

Bortezomib: batch number: 20191102, made by CSPC Zhongqi Pharmaceutical Technology (Shijiazhuang) Co., Ltd, dissolved in normal saline containing 2% DMSO immediately prior to use.
Butylphthalide (oral administration grade), 10 kg/bottle, batch number: 518180803, made by CSPC NBP Pharmaceutical Co., Ltd, diluted in vegetable oil to a target concentration immediately prior to use.
Duloxetine hydrochloride, batch number: QRQYD-JN, made by TCI (Shanghai) Development Co., Ltd, prepared

with normal saline containing 2% DMSO immediately prior to use.

3. Experimental process

3.1 Model preparation and administration method

In this experiment, male SD rats were administered with bortezomib at a dose of 0.3 mg/kg with an administration volume of 5 mL/kg, and were intraperitoneally injected on days 0, 2, 4, 6, 8, 10, and 13 for modeling.

Three doses (3, 10, and 30 mg/kg bid) of NBP were used. The first dose of NBP was administered by transoral gavage 1 day prior to the Bortezomib modeling. During the Bortezomib administration and modeling, the first dose of NBP was administered 1 h prior to the Bortezomib administration every day. The second dose of NBP was administered with an interval of ≥4 h from the first dose, with an administration volume of 10 mL/kg, and was administered for 21 consecutive days.

The normal group was given normal saline containing 2% DMSO at the same frequency and with the same administration method as Bortezomib, and was given vegetable oil at the same frequency and with the same administration method as NBP. (The modeling and administration time are shown in Fig. 11. The animal grouping, the administration method, and the doses are detailed in Table 14).

Table 14 Animal Grouping and Dose List

| Group | Number of animals | Drug | Dose (mg/kg) | Administration route |
|---|---|---|---|---|
| Normal control group | 12 | Vegetable oil/normal saline containing 2% DMSO | 0/0 | p.o./i.p. |
| Model group | 12 | Vegetable Oil/Bortezomib | 0/0.3 | p.o./i.p. |
| Low-dose group of NBP | 12 | NBP/Bortezomib | 3/0.3 | p.o./i.p. |
| Moderate-dose group of NBP | 12 | NBP/Bortezomib | 10/0.3 | p.o./i.p. |
| High-dose group of NBP | 12 | NBP/Bortezomib | 30/0.3 | p.o./i.p. |
| Positive drug duloxetine group | 12 | Duloxetin/Bortezomib | 15/0.3 | p.o./i.p. |

3.2 Grouping

Based on the thermal pain threshold baseline values and the body weights of the rats, the rats with the baseline values ranging from 3 to 6 s were selected, and were equally grouped in accordance with Table 14 in 3.1.

3.3 Observation indexes

(1) Body weight: all animals were weighed once prior to the experiment, and were weighed at set time every day after the commencement of administration.

(2) Thermal pain threshold: measurements were performed on days 0, 7, 14, and 21.
Method: IITC Life Science Electronic Von Frey Anesthesio meter was used and the intensity of the light source was adjusted to obtain a thermal pain threshold baseline of about 3-6 s. In this case, the light intensity was 58%, and the cutoff value was set as 10 s. The rats were placed in a plastic compartment on a glass plate, to make the planta centers of the rats be exposed to the light source. The time of reflexive withdrawal, i.e., the thermal pain threshold, of the rats was recorded, and was measured once for each of the left foot and the right foot of each rat, the measurement was repeated three times with each interval therebetween ≥15 min, to compute a mean value of the 6 thermal pain thresholds of each rat.

(3) Mechanical stimulus pain threshold: measurements were performed on days -1, 6, 13, and 20.
Method: the rats were placed in a plastic compartment on a wire mesh, left to stand still for 15 min, stimulated using a stimulation probe with a diameter of 0.4 mm of the IITC Life Science Electronic Von Frey Anesthesio meter at the planta centers of the rats by gradually increasing the force until reflexive withdrawal of the rats. The maximum value displayed by the instrument at this time, i.e., the mechanical pain threshold, was recorded. In each experiment, the left and right feet of each rat were measured three times with each interval therebetween ≥5 min, to compute a mean value of the 6 mechanical pain thresholds of each rat.

4. Results

4.1 Influence on body weight

On day 21 of the experiment, compared with the normal control group, the body weights of the rats in the model group were significantly decreased ($P<0.05$); and compared with the model group, the body weights of the rats in the three dose groups of NBP (3, 10, and 30 mg/kg bid) were not obviously changed, as detailed in Fig. 12.

4.2 Influence on thermal pain threshold

On day 21 of the experiment, compared with the normal control group, the thermal pain threshold of the model group was significantly decreased; and compared with the model group, the decrease of the thermal pain threshold of the model rats in the group of NBP 3, 10, and 30 mg/kg bid can be significantly reversed, as detailed in Fig. 13.

4.3 Influence on mechanical stimulus pain threshold

On day 20 of the experiment, compared with the normal control group, the mechanical stimulus pain threshold of the model group was significantly decreased; and compared with the model group, the decrease of the mechanical stimulus pain threshold of the model rats in the group of NBP 10 and 30 mg/kg bid can be significantly reversed, as detailed in Fig. 14.

5. Conclusions

Under the conditions of this experiment, NBP can dose-dependently effectively relieve the symptoms of Bortezomib-induced peripheral neuropathy in the CIPN model of rat.

Example 7: Experiment for influence of NBP on in vitro anti-tumor activity of Bortezomib

1. Cell and culture conditions

Table 15 Cell and Essential Culture Medium

| Cell strains | Source | Histological source | Culture medium |
|---|---|---|---|
| MM.1S | Nanjing Cobioer Biosciences Co., Ltd | Human multiple myeloma cells | RPMI Medium 1640 containing 10% FBS |
| Jeko-1 | Shanghai Institutes for Biological Sciences, Chinese Academy of Sciences | Human mantle cell lymphoma cells | RPMI Medium 1640 containing 20% FBS |
| HT29 | Shanghai Institutes for Biological Sciences, Chinese Academy of Sciences | Human colon cancer cells | McCoy's 5A containing 10% FBS |
| A549 | ATCC | Human non-small cell lung cancer cells | RPMI Medium 1640 containing 10% FBS |
| DU145 | Institute of Basic Medical Sciences, Chinese Academy of Medical Sciences | Human prostate cancer cells | RPMI Medium 1640 containing 10% FBS |

The above cell culture conditions were 37 °C and 5% $CO_2$.

2. Experimental objective

To investigate whether NBP affects the function of Bortezomib in the in vitro inhibition of tumor cell proliferation.

3. Drugs

Bortezomib (bulk drug), made by CSPC Zhongqi Pharmaceutical Technology (Shijiazhuang) Co., Ltd., batch number: 20190802

Butylphthalide (oral administration grade), 10 kg/bottle, made by CSPC NBP Pharmaceutical Co., Ltd., batch number: 518180803

4. Experimental design

This experiment includes a monotherapy group of Bortezomib, a combined therapy group (Bortezomib/NBP), and a monotherapy group of NBP. Different drug concentration gradients were set for each group.

Monotherapy group of Bortezomib: ① MM.1S, Jeko-1, and HT29 cells: Bortezomib was 1.5× serially diluted from an initial concentration of 20 nM to 8 concentrations: 20, 13.33, 8.89, 5.93, 3.95, 2.63, 1.76, and 1.17 nM, respectively; ② A549 cells: Bortezomib was 2× serially diluted from an initial concentration of 200 nM to 8 concentrations: 200, 100, 50, 25, 12.5, 6.25, 3.13, and 1.56 nM, respectively; and ③ DU145 cells: Bortezomib was 2× serially diluted from an initial concentration of 100 nM to 8 concentrations: 100, 50, 25, 12.5, 6.25, 3.13, 1.56, and 0.78 nM, respectively.

Bortezomib/NBP group: the final concentration of NBP was 30 μM, and the concentration gradient of Bortezomib was the same as the final concentration of the monotherapy group of Bortezomib.

Monotherapy group of NBP: NBP was 2× serially diluted from an initial concentration of 480 μM to 8 concentrations: 480, 240, 120, 60, 30, 15, 7.5, and 3.75 μM, respectively.

The action time of the drug was 72 h.

5. Experimental process

A certain number of conventionally cultured cells in logarithmic growth phase were inoculated in a 96-well plate with 100 μL per well. For suspension cells MM.1S and Jeko-1, on the day of inoculation, 100 μL of a culture solution containing different concentration gradients of Bortezomib was added into each well of the Bortezomib group, 100 μL of a culture solution containing different concentration gradients of Bortezomib and 30 μM NBP was added into each well of the combined therapy group of Bortezomib/NBP, and 100 μL of a culture solution containing different concentration gradients of NBP was added into each well of the monotherapy group of NBP. For adherent cells HT29, A549, and DU145, the above drugs were added 24 h after adherence. 3 replicate wells were set for each concentration of each drug, and blank wells (only culture medium was present, and no tumor cells were inoculated) and normal wells (a culture medium inoculated with tumor cells, drug concentration was 0) were set. The drugs functioned for 72 h, then MTT working solution (5 mg/mL) was added at 20 μL per well; after functioning at 37 °C for 4 h, the supernatant was removed, 150 μL of DMSO (analytically pure) was added; the mixture was fully mixed using a microplate oscillator, the plate was wiped clean, and the optical density (OD) at 550 nm was measured using a microplate reader.

The cell growth inhibition rate was computed using the following equation:

$$\text{Inhibition rate (\%)} = (\text{OD value}_{\text{normal well}} - \text{OD value}_{\text{administration well}}) / (\text{OD value}_{\text{normal well}} - \text{OD value}_{\text{blank well}}) \times 100\%$$

Based on the inhibition rate of each concentration, the median inhibitory concentration $IC_{50}$ of the drug was computed using SPSS19.0.

The experiment was repeated three times, and the $IC_{50}$ value was expressed as the mean ± standard deviation of the results of the 3 experiments.

6. Results

The results showed that both the monotherapy group of Bortezomib and the combined therapy group of Bortezomib/NBP had obvious inhibitory effect on the in vitro proliferation of MM.1S, Jeko-1, HT29, A549, and DU145, and mean $IC_{50}$ values thereof were in the range from 1 to 20 nM, suggesting that NBP did not affect the function of Bortezomib

in the in vitro inhibition of tumor cell proliferation, as detailed in Fig. 16.

Table 16 $IC_{50}$ Values (nM) of Bortezomib and Combined Therapy of Bortezomib/NBP for 5 Cell Strains

| | Bortezomib | | | | Bortezomib/NBP | | | |
|---|---|---|---|---|---|---|---|---|
| | 1st dose | 2nd dose | 3rd dose | Mean ± SD | 1st dose | 2nd dose | 3rd dose | Mean ± SD |
| MM.1S | 2.55 | 2.94 | 2.99 | 2.83±0.24 | 2.55 | 3.25 | 2.99 | 2.93±0.35 |
| Jeko-1 | 4.83 | 3.51 | 4.47 | 4.27±0.68 | 5.82 | 3.45 | 4.60 | 4.62±1.19 |
| HT29 | 4.43 | 4.97 | 5.65 | 5.02±0.61 | 4.67 | 5.56 | 5.72 | 5.32±0.56 |
| A549 | 11.82 | 13.31 | 10.56 | 11.89±1.38 | 9.01 | 11.60 | 10.92 | 10.51±1.34 |
| DU145 | 12.79 | 11.88 | 17.13 | 13.94±2.80 | 9.59 | 10.39 | 15.32 | 11.77±3.10 |

The monotherapy of NBP does not have obvious inhibitory effect on the in vitro proliferation of human multiple myeloma cells (MM.1S), human mantle cell lymphoma cells (Jeko-1), human colon cancer cells (HT29), human lung cancer cells (A549), and human prostate cancer cells (DU145). The inhibition rate of 30 μM NBP alone on the proliferation of 5 cell strains is detailed in Table 17.

Table 17 Inhibition Rate (%) of 30 μM NBP Alone on Proliferation of 5 Cell Strains

| Cell type | Inhibition rate (%, 30 μM NBP) | | | |
|---|---|---|---|---|
| | First dose | Second dose | Third dose | Mean ± SD |
| MM.1S | 0.16 | 14.08 | 22.08 | 12.11±11.09 |
| Jeko-1 | -7.67 | -18.83 | -8.11 | -11.54±6.32 |
| HT29 | 9.98 | 8.27 | 13.73 | 10.66±2.79 |
| A549 | -11.08 | 9.68 | 4.74 | 1.11±10.84 |
| DU145 | -2.22 | 9.44 | 4.87 | 4.03±5.88 |

Conclusions: NBP does not affect the inhibitory effect of Bortezomib on the in vitro proliferation of human multiple myeloma cells (MM.1S), human mantle cell lymphoma cells (Jeko-1), human colon cancer cells (HT29), human lung cancer cells (A549), and human prostate cancer cells (DU145).

Example 8: Experiment for influence of NBP on in vivo anti-tumor efficacy of Bortezomib

1. Experimental system

1.1 Experimental animals

42 female Nu/Nu mice, 18-20 g.

1.2 Cell strains

MM.1S cells (human multiple myeloma cells), purchased from Nanjing Cobioer Biosciences Co., Ltd.

2. Experimental objective

A xenograft model of MM.1S cells of the Nu/Nu mice was used in this experiment to validate the influence of NBP on the antitumor efficacy and PK behavior of Bortezomib.

3. Drugs

Bortezomib (bulk drug): manufacturer: CSPC Zhongqi Pharmaceutical Technology (Shijiazhuang) Co., Ltd, batch number: 20191102; dissolved in normal saline containing 2% DMSO immediately prior to use.

Butylphthalide (oral administration grade), 10 kg/bottle, manufacturer: CSPC NBP Pharmaceutical Co., Ltd, batch number: 518180803, diluted in vegetable oil to a target concentration immediately prior to use.
Palbociclib (bulk drug), manufacturer: Shanghai Fangnan Biotechnology Co., Ltd, batch number: AL-0127-API-1811001; prepared with a solution of propylene glycol and 20% hydroxypropyl-β-cyclodextrin at a ratio of 5:95 to a solution having a target concentration immediately prior to use.

4. Experimental process

4.1 Model preparation

After in vitro resuscitation and passage of MM.1S cells to a sufficient amount, the cells were counted by a microscope, and diluted with a serum-free medium to adjust the cell count to about $1\times10^8$ cells/mL. The cell suspension was kept in an ice-water bath.
The MM.1S cell suspension was extracted by a sterile syringe and inoculated into the subcutaneous tissue of the forelimb axilla of the Nu/Nu mice. The inoculation volume was 0.1 mL/mouse, containing about $1.0\times10^7$ tumor cells, to prepare MM.1S xenograft tumor model of the Nu/Nu mice.

4.2 Administration method

Monotherapy group of Bortezomib: administered by intraperitoneal injection at 0.5 mg/kg, biw (on days 0, 3, 7, 10, and 15);

Monotherapy group of NBP: administered by transoral gavage at 60 mg/kg, bid (the administration was commenced on day 0), and the second dose of NBP was administered with an interval from the first dose of ≥4 h;

Monotherapy group of Palbociclib: intragastric administration at 70 mg/kg, qd (the administration was commenced on day 0);

Combined therapy group of NBP/Bortezomib: the administration frequency and dose were the same as the frequency and dose of the monotherapy group of NBP and the monotherapy group of Bortezomib, respectively.

Combined therapy group of Bortezomib/Palbociclib: the administration frequency and dose were the same as the frequency and dose of the monotherapy group of Bortezomib and the monotherapy group of Palbociclib, respectively.
Combined therapy group of NBP/Bortezomib/Palbociclib: the administration frequency and dose were the same as the frequency and dose of the monotherapy group of NBP, the monotherapy group of Bortezomib, and the monotherapy group of Palbociclib, respectively.
The solvent group was given the solvent at the same frequency and with the same administration method as Bortezomib and NBP.
The administration period was 16 days, and the administration volume was 10 mL/kg.

4.3 Observation indexes and evaluation indexes

4.3.1 Observation indexes

(1) General state observation: all animals were observed once per day during the experimental period, and the abnormality and behavioral changes of their body parts were recorded.

(2) Body weight: all animals were weighed once prior to the experiment, and animals having suitable body weights were selected for the experiment. Animals were weighed at set time once per day after the commencement of administration.

(3) Death and near death: the death time of dead animals was recorded, and dying animals should be observed more frequently to determine the death time.

4.3.2 Tumor volume evaluation

After animals were grouped, the long diameter and the short diameter of the tumor were measured twice every week.

(1) Tumor volume: $V=1/2\times A\times B^2$

(2) Relative tumor volume: $$RTV=\frac{TVn}{TV0d}$$

(3) Relative tumor volume proliferation rate:

$$T/C\%=\frac{\text{Therapy group RTVxnd}}{\text{Solvent group RTVxnd}}\times 100\%$$

(4) Tumor growth inhibition rate:

$$TGI\%=[1-\frac{(TV_Xn-TV_{X0})}{(TV_Mn-TV_{M0})}]\times 100\%$$

Note: V: tumor volume; A: tumor length; B: tumor width; RTV: relative tumor volume; $TV_{nd}$: tumor volume on day n; $TV_{0d}$: tumor volume on day 0; $RTV_{xnd}$: mean relative tumor volume on day n; $TV_{Xn}$: mean tumor volume of the therapy group on day n; $TV_{X0}$: mean tumor volume of the therapy group on day 0; $TV_{Mn}$: mean tumor volume of the solvent group on day n; and $TV_{M0}$: mean tumor volume of the solvent group on day 0

4.3.3 Tumor weight

At the end of the experiment, after the animals were euthanized by $CO_2$ asphyxiation, the tumors were excised and weighed.

Tumor weight inhibition rate% = (1−tumor weight in therapy group/tumor weight in solvent group) × 100%

4.3.4 PK blood sampling and detection

Before administration of the last dose of Bortezomib (0 h), and 5 min, 15 min, 30min, 1 h, 2 h, 4 h, 8 h, 24 h after the administration, blood was sampled (as detailed in Table 8) (except that blood was sampled from the mice in the solvent group only at 0 h), anticoagulated with heparin, and centrifuged to separate plasma, which was stored at -80 °C for later use. Bortezomib and butyphthalide in the plasma were determined by protein precipitation-LC/MS/MS.

Table 18 List of Blood Sampling Time of Mice in Each Group

| Group | Animal No. | Blood sampling time |
|---|---|---|
| Solvent group | 1-6 | 0 h |
| Therapy groups | 1-6 | 0, 5 min, 15 min, 30 min, 1 h, 2 h, 4 h, 8 h, 24 h |

5. Experimental results

5.1 Influence on body weight

As can be seen from the body weight data, there was no significant difference in the body weights of the mice in each group ($P$>0.05), indicating that none of Bortezomib, Palbociclib, and NBP had influence on the body weights of the mice, as detailed in Fig. 15.

5.2 Influence on tumor volume

At the end of the experiment, compared with the monotherapy group of Bortezomib, during the combined therapy of NBP/Bortezomib, NBP did not have obvious negative influence on the tumor inhibition efficacy of Bortezomib; and compared with the combined therapy group of Bortezomib/Palbociclib, in the combined therapy group of NBP/Bortezomib/Palbociclib, NBP did not have obvious negative influence on the antitumor efficacy of combined therapy of Bortezomib/Palbociclib, either. The tumor volume of each group is detailed in Fig. 16, and the body weight, TV, RTV, TGI%, T/C%, tumor weight, etc. at the end of the experiment (day 14) are detailed in Table 19.

Table 19 Summary of Various Index Parameters of NU/NU Mice on Day 14 of the Experiment (mean ±SD, n=6)

| Index | Solvent | Bor 0.5mg/kg | Pal 70 mg/kg | Bor/Pal 0.5/70 mg/kg | NBP/Bor/Pal 60/0.5/70 mg/kg | NBP/Bor 60/0.5mg/kg | NBP 60 mg/kg |
|---|---|---|---|---|---|---|---|
| Body weight(g) | 24.4±1.8 | 24.7±1.2 | 23.9±1.7 | 24.0±0.9 | 24.1±2.1 | 23.8±1.3 | 24.9±1.2 |
| TV ($mm^3$) | 1220.41625 .3 | 1124.8±260 .5 | 605.4±388. 7 | 436.7±178. 3 | 420.61178.9 | 876.9±300.8 | 938.11451.9 |
| RTV | 9.812.1 | 9.6±2.8 | 4.3±1.5 | 3.4±0.9 | 3.3±0.6 | 7.2±2.8 | 7.2±2.0 |
| TGI% | --- | 8.7 | 56.3 | 71.7 | 73.1 | 31.3 | 25.8 |
| T/C% | | 98.0 | 44.4 | 34.9 | 34.2 | 73.9 | 73.8 |
| Tumor weight | 1.648±0.67 8 | 1.282±0.20 6 | 0.550±0.39 2 | 0.391±0.17 6 | 0.413±0.227 | 1.086±0.341 | 1.040±0.398 |
| Tumor weight inhibition rate (%) | --- | 22.2 | 66.6 | 76.3 | 75.0 | 34.1 | 36.9 |
| Note: Bor represents Bortezomib, and Pal represents Palbociclib. | | | | | | | |

Conclusions: in this experiment, NBP did not have obvious influence on the antitumor efficacy of Bortezomib and of the combined therapy of Bortezomib/Palbociclib.

5.3 Influence on PK

The results showed that the Bortezomib exposure doses in the bodies of the mice in the Bortezomib group, the NBP/Bortezomib group, the Bortezomib/Palbociclib group, and the NBP/Bortezomib/Palbociclib group were substantially consistent, suggesting that NBP did not have obvious influence on the in vivo pharmacokinetic behaviors of the monotherapy of Bortezomib and the combined therapy of Bortezomib/Palbociclib. The main pharmacokinetic parameters are detailed in Table 20, Table 21, and Table 22.

Table 20 Summary of Main Pharmacokinetic Parameters of Bortezomib in Plasma of NU/NU Mice (mean ± SD, n=6)

| Parameters (unit) | Bor 0.5 mg/kg | Bor/Pal 0.5/70 mg/kg | NBP/Bor/Pal 60/0.5/70 mg/kg | NBP/Bor 60/0.5mg/kg |
|---|---|---|---|---|
| $C_{max}$ (ng/mL) | 113±8 | 114±17 | 128±16 | 126±37 |
| $AUC_{0-t}$ (h*ng/mL) | 290±104 | 322±57 | 234±66 | 264±75 |
| $t_{1/2}$(h) | 56.6±1.16 | 40.2±14.8 | 65.6±66.2 | 59.1163.5 |
| Vd (L/kg) | 14.2±3.2 | 24.2±6.4 | 37.2±11.6 | 24.2±7.1 |
| Cl(L/h/kg) | 1.81±0.56 | 0.44±0.09 | 0.86±0.67 | 0.97±0.91 |
| $MRT_{0-t}$ (h) | 5.9±0.8 | 12.7±2.2 | 10.5±0.9 | 9.5±4.2 |

Table 21 Summary of Main Pharmacokinetic Parameters of NBP in Plasma of NU/NU Mice (mean ± SD, n=6)

| Parameters (unit) | NBP/Bor/Pal 60/0.5/70 mg/kg | NBP/Bor 60/0.5 mg/kg | NBP 60 mg/kg |
|---|---|---|---|
| $C_{max}$ (ng/mL) | 145±108 | 125±74 | 117±88 |
| $AUC_{0-t}$(h*ng/mL) | 242±259 | 433±166 | 236±100 |
| $t_{1/2}$ (h) | 4.91±2.52 | 10.9±3.2 | 10.1±9.7 |
| Vd (L/kg) | 1979±1486 | 1723±699 | 14451298 |
| Cl(L/h/kg) | 258±124 | 112±36 | 173±104 |
| $MRT_{0-t}$(h) | 2.1±0.7 | 7.5±1.2 | 3.1±0.6 |
| Note: In Tables 20 and 21, Bor represents Bortezomib, Pal represents Palbociclib, $C_{max}$ represents a maximum blood concentration, $AUC_{0-t}$ represents the area under the drug-time curve from 0 to t, $t_{1/2}$ represents the half-life, Vd represents the apparent volume of distribution, Cl represents the clearance rate, and $MRT_{0-t}$ represents the mean residence time from 0 to t. | | | |

Table 22 Results of Comparison between Mean Plasma Exposures of Bortezomib and NBP in Combined Therapy Group and Monotherapy Group

| Group | Ratio to monotherapy group of Bortezomib | | | |
|---|---|---|---|---|
| | $C_{max}$ | $AUC_{0-t}$ | LN (Cmax) | LN ($AUC_{0-t}$) |
| Bor/Pal 0.5/70 mg/kg | 1.01 | 1.11 | 1.00 | 1.02 |
| NBP/Bor/Pal 60/0.5/70 mg/kg | 1.13 | 0.81 | 1.05 | 0.96 |
| NBP/Bor 60/0.5mg/kg | 1.12 | 0.91 | 1.02 | 0.98 |

(continued)

| Group | Ratio to monotherapy group of NBP | | | |
|---|---|---|---|---|
| | $C_{max}$ | $AUC_{0-t}$ | LN (Cmax) | LN ($AUC_{0-t}$) |
| NBP/Bor/Pal 60/0.5/70 mg/kg | 1.24 | 1.03 | 1.05 | 1.00 |
| NBP/Bor 60/0.5 mg/kg | 1.08 | 1.83 | 1.02 | 1.11 |
| Note: Bor represents Bortezomib, and Pal represents Palbociclib. | | | | |

The ratios of $C_{max}$ and $AUC_{0-t}$ of Bortezomib in the combined therapy groups of Bortezomib/Palbociclib, the combined therapy groups of NBP/Bortezomib/Palbociclib, and the combined therapy groups of NBP/Bortezomib to those in the monotherapy group of Bortezomib were 1.01-1.13 and 0.81-1.11, respectively. Upon logarithmic transformation of $C_{max}$ and $AUC_{0-t}$, the ratios of the combined therapy groups of Bortezomib/Palbociclib, the combined therapy groups of NBP/Bortezomib/Palbociclib, and the combined therapy groups of NBP/Bortezomib to the monotherapy group of Bortezomib were 1.00-1.05 ($LNC_{max}$) and 0.96-1.02 ($LNAUC_{0-t}$), respectively, therefore the in vivo exposures of Bortezomib in the monotherapy group and the combined therapy groups were substantially consistent.

The ratios of $C_{max}$ and $AUC_{0-t}$ of NBP in the combined therapy groups of NBP/Bortezomib/Palbociclib and the combined therapy groups of NBP/Bortezomib to those in the monotherapy group of NBP were 1.08-1.24 and 1.03-1.83, respectively. Upon logarithmic transformation of $C_{max}$ and $AUC_{0-t}$, the ratios of the combined therapy groups of NBP/Bortezomib/Palbociclib and the combined therapy groups of NBP/Bortezomib to the monotherapy group of NBP were 1.02-1.05 ($LNC_{max}$) and 1.00-1.11 ($LNAUC_{0-t}$), respectively. Due to the obvious individual differences in mice after intragastric administration of NBP, a small number of mice were arranged in each experimental group. The ratio of the exposure level of the combined therapy group to the exposure level of the monotherapy group after logarithmic transformation showed that the in vivo exposure of the monotherapy group of NBP was not obviously different from the in vivo exposure of the combined therapy group.

Conclusions: in this experiment, NBP substantially did not affect the pharmacokinetic behavior of Bortezomib in mice, and Bortezomib substantially did not affect the pharmacokinetic behavior of NBP in mice.

## Claims

1. Use of butylphthalide or an optical isomer, a prodrug, a deuterated product, a metabolite, a ring-opening product, or a salt of the ring-opening product thereof in the preparation of a drug for preventing, relieving, or treating a peripheral neuropathy.

2. The use according to claim 1, wherein the peripheral neuropathy is preferably a drug-induced peripheral neuropathy, and more preferably a chemotherapeutic drug-induced peripheral neuropathy.

3. The use according to any one of claims 1 to 2, wherein the preventing, relieving, or treating a peripheral neuropathy includes, but is not limited to, preventing, relieving, or treating chemotherapeutic drug-induced paresthesia and dyskinesia, such as paresthesia of pain and heat, or impaired motor coordination.

4. The use according to any one of claims 1 to 3, wherein the drug comprises a therapeutically effective amount of butylphthalide or the optical isomer, the prodrug, the deuterated product, the metabolite, the ring-opening product, or the salt of the ring-opening product thereof.

5. The use according to any one of claims 1 to 4, wherein the drug is prepared into a clinically acceptable formulation, such as an oral formulation, an injectable formulation, a topical formulation, or an external formulation, and preferably an oral formulation and an injectable formulation.

6. The use according to any one of claims 1 to 5, wherein the drug is a single dose form or a divided dose form.

7. The use according to any one of claims 1 to 6, wherein the drug is an oral formulation; and the oral formulation comprises from about 1 mg to about 1,000 mg, and preferably from about 1 mg to about 500 mg, or from about 1 mg to about 300 mg, or from about 1 mg to about 200 mg, or from about 5 mg to about 180 mg, or from about 10 mg to about 150 mg, or from about 30 mg to about 120 mg, or from about 50 mg to about 120 mg, or from about

80 mg to about 120 mg, or from about 90 mg to about 110 mg, or about 100 mg, of butylphthalide or the optical isomer, the prodrug, the deuterated product, the metabolite, the ring-opening product, or the salt of the ring-opening product thereof, on a basis of butylphthalide.

**8.** The use according to claim 7, wherein a daily dose of the oral formulation is from about 1 mg to about 10,000 mg, preferably from about 10 mg to about 5,000 mg, or from about 20 mg to about 3,000 mg, or from about 30 mg to about 2,000 mg, or from about 50 mg to about 1,500 mg, or from about 70 mg to about 1,200 mg, or from about 100 mg to about 1,000 mg, or from about 200 mg to about 900 mg, or from about 300 mg to about 800 mg, or from about 400 mg to about 700 mg, or from about 500 mg to about 600 mg, or from about 60 mg to about 800 mg, or from about 60 mg to about 600 mg, or from about 100 mg to about 800 mg, or from about 100 mg to about 600 mg, or from about 200 mg to about 600 mg, or from about 200 mg to about 800 mg, or from about 300 mg to about 600 mg, or from about 400 mg to about 600 mg, or from about 400 mg to about 800 mg, on a basis of butylphthalide.

**9.** The use according to claim 8, wherein the oral formulation is administered once per day by administering from about 60 mg to about 800 mg of the oral formulation of butylphthalide or the optical isomer, the prodrug, the deuterated product, the metabolite, the ring-opening product, or the salt of the ring-opening product thereof, e.g., administering about 60 mg, about 100 mg, about 200 mg, about 300 mg, about 400 mg, about 500 mg, about 600 mg, about 700 mg, or about 800 mg of the oral formulation each time, on a basis of butylphthalide; or, the oral formulation is administered twice per day by administering from about 30 mg to about 400 mg of the oral formulation of butylphthalide or the optical isomer, the prodrug, the deuterated product, the metabolite, the ring-opening product, or the salt of the ring-opening product thereof each time, e.g., administering about 30 mg, about 50 mg, about 100 mg, about 150 mg, about 200 mg, about 250 mg, about 300 mg, about 350 mg, or about 400 mg of the oral formulation each time, on a basis of butylphthalide; or, the oral formulation is administered thrice per day by administering from about 20 mg to about 300 mg of the oral formulation of butylphthalide or the optical isomer, the prodrug, the deuterated product, the metabolite, the ring-opening product, or the salt of the ring-opening product thereof, e.g., administering about 20 mg, about 50 mg, about 100 mg, about 150 mg, about 200 mg, about 250 mg, or about 300 mg of the oral formulation each time, on a basis of butylphthalide.

**10.** The use according to any one of claims 1 to 6, wherein the drug is an injectable formulation; and the injectable formulation comprises from about 0.001 mg/mL to about 100 mg/mL, and preferably from about 0.005 mg/mL to about 50 mg/mL, or from about 0.01 mg/mL to about 10 mg/mL, or from about 0.1 mg/mL to about 5 mg/mL, or from about 0.1 mg/mL to about 3 mg/mL, or from about 0.1 mg/mL to about 1 mg/mL, or from about 0.12 mg/mL to about 0.80 mg/mL, or from about 0.15 mg/mL to about 0.50 mg/mL, and more preferably from about 0.20 mg/mL to about 0.40 mg/mL, or from about 0.20 mg/mL to about 0.30 mg/mL, or about 0.25 mg/mL, of butylphthalide or the optical isomer, the prodrug, the deuterated product, the metabolite, the ring-opening product, or the salt of the ring-opening product thereof, on a basis of butylphthalide.

**11.** The use according to claim 10, wherein a daily dose of the injectable formulation is from about 1 mg to about 1,000 mg, preferably from about 5 mg to about 500 mg, or from about 10 mg to about 300 mg, or from about 15 mg to about 200 mg, or from about 20 mg to about 150 mg, or from about 25 mg to about 120 mg, or from about 30 mg to about 100 mg, or from about 35 mg to about 90 mg, or from about 40 mg to about 80 mg, or from about 45 mg to about 70 mg, or from about 50 mg to about 60 mg, or from about 1 mg to about 100 mg, or from about 2 mg to about 80 mg, or from about 5 mg to about 75 mg, or from about 10 mg to about 50 mg, or from about 15 mg to about 50 mg, or from about 20 mg to about 50 mg, or from about 25 mg to about 75 mg, or from about 25 mg to about 50 mg, on a basis of butylphthalide.

**12.** The use according to claim 11, wherein the injectable formulation is administered once per day by administering from about 1 mg to about 100 mg, and preferably about 1 mg, about 2 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg, of the injectable formulation of butylphthalide or the optical isomer, the prodrug, the deuterated product, the metabolite, the ring-opening product, or the salt of the ring-opening product thereof each time, on a basis of butylphthalide; or, the injectable formulation is administered twice per day by administering from about 1 mg to about 50 mg, and preferably about 1 mg, about 2 mg, about 2.5 mg, about 5 mg, about 10 mg, about 15 mg, about 20 mg, about 25 mg, about 30 mg, about 35 mg, about 40 mg, about 45 mg, or about 50 mg, of butylphthalide or the optical isomer, the prodrug, the deuterated product, the metabolite, the ring-opening product, or the salt of the ring-opening product thereof each time.

**13.** The use according to any one of claims 1 to 12, wherein the chemotherapeutic drug includes, but is not limited to, one or more of following drugs: (1) a chemotherapeutic drug acting on a microtubule system or an anti-mitotic chemotherapeutic drug, including: a taxane drug, such as paclitaxel, docetaxel or the like; or a vinca alkaloid drug, such as vincristine, vinblastine or the like; or an epothilone drug, such as ixabepilone or the like; or a protease inhibitor drug, such as bortezomib or the like; or (2) a chemotherapeutic drug that interferes with DNA synthesis, including a platinum drug, such as cisplatin, carboplatin, oxaliplatin or the like; or (3) an immunomodulator drug, such as thalidomide, lenalidomide or the like.

**14.** The use according to any one of claims 1 to 13, wherein the butylphthalide or the optical isomer, the prodrug, the deuterated product, the metabolite, the ring-opening product, or the salt of the ring-opening product thereof may be used in the preparation of the drug in combination with one or more of other drugs for treating peripheral neuropathy; and preferably, the other drugs for treating peripheral neuropathy are duloxetine or a salt thereof, monosialotetrahexosyl ganglioside sodium.

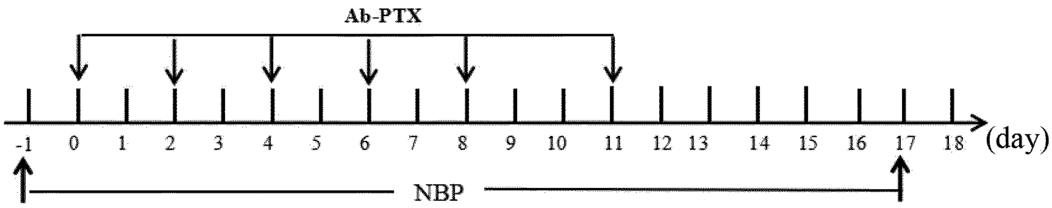


Fig. 1

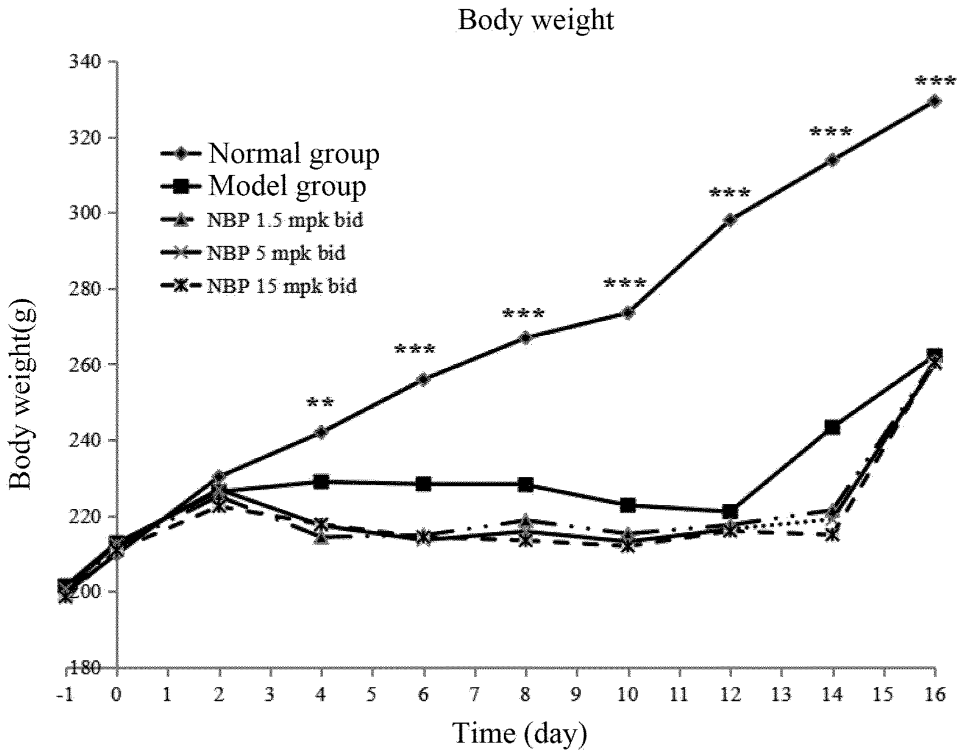


Fig. 2

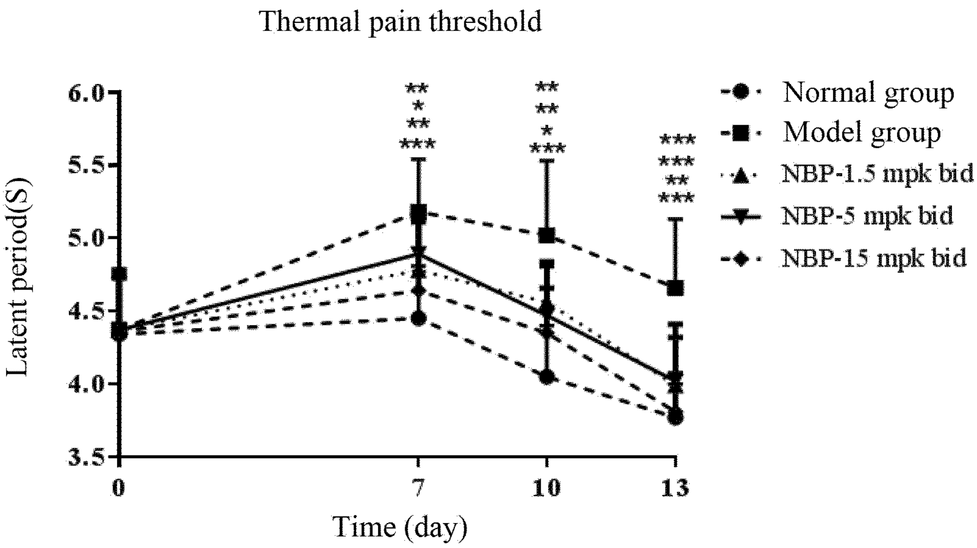


Fig. 3

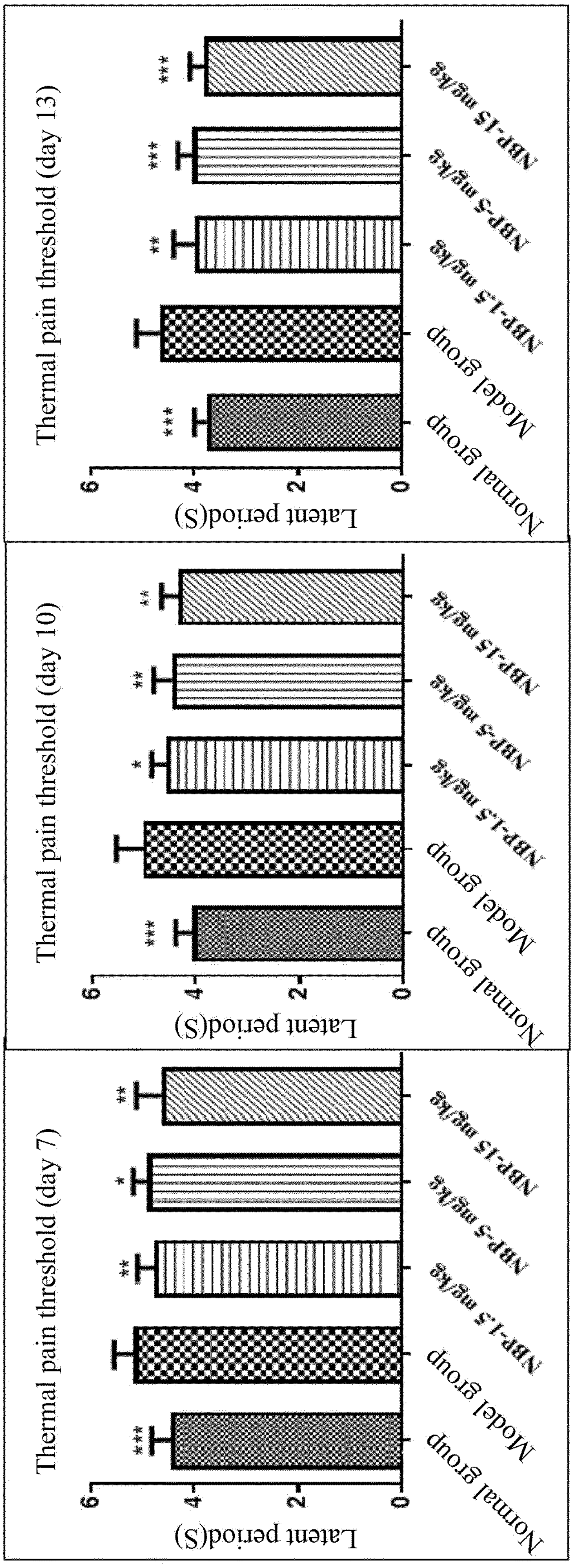
Thermal pain threshold (day 7)
Thermal pain threshold (day 10)
Thermal pain threshold (day 13)
Latent period(S)
0
2
4
6
***
**
*
Normal group
Model group
NBP-1.5 mg/kg
NBP-5 mg/kg
NBP-15 mg/kg

Fig. 4

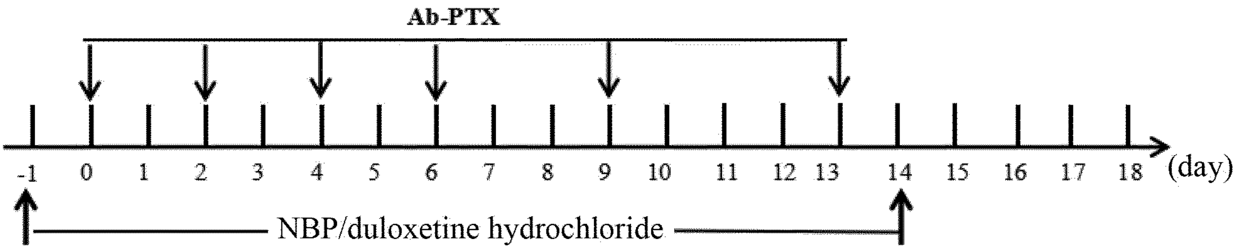
Ab-PTX
-1
0
1
2
3
4
5
6
7
8
9
10
11
12
13
14
15
16
17
18
(day)
NBP/duloxetine hydrochloride

Fig. 5

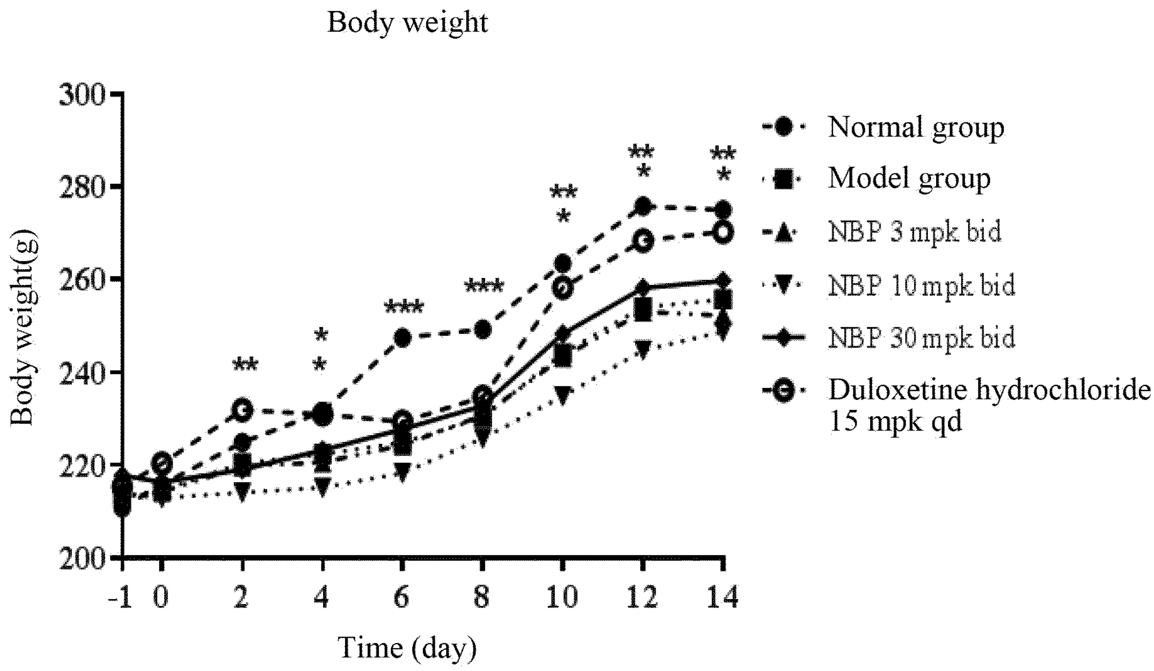
Body weight
Body weight(g)
300
280
260
240
220
200
-1
0
2
4
6
8
10
12
14
Time (day)
Normal group
Model group
NBP 3 mpk bid
NBP 10 mpk bid
NBP 30 mpk bid
Duloxetine hydrochloride
15 mpk qd

Fig. 6

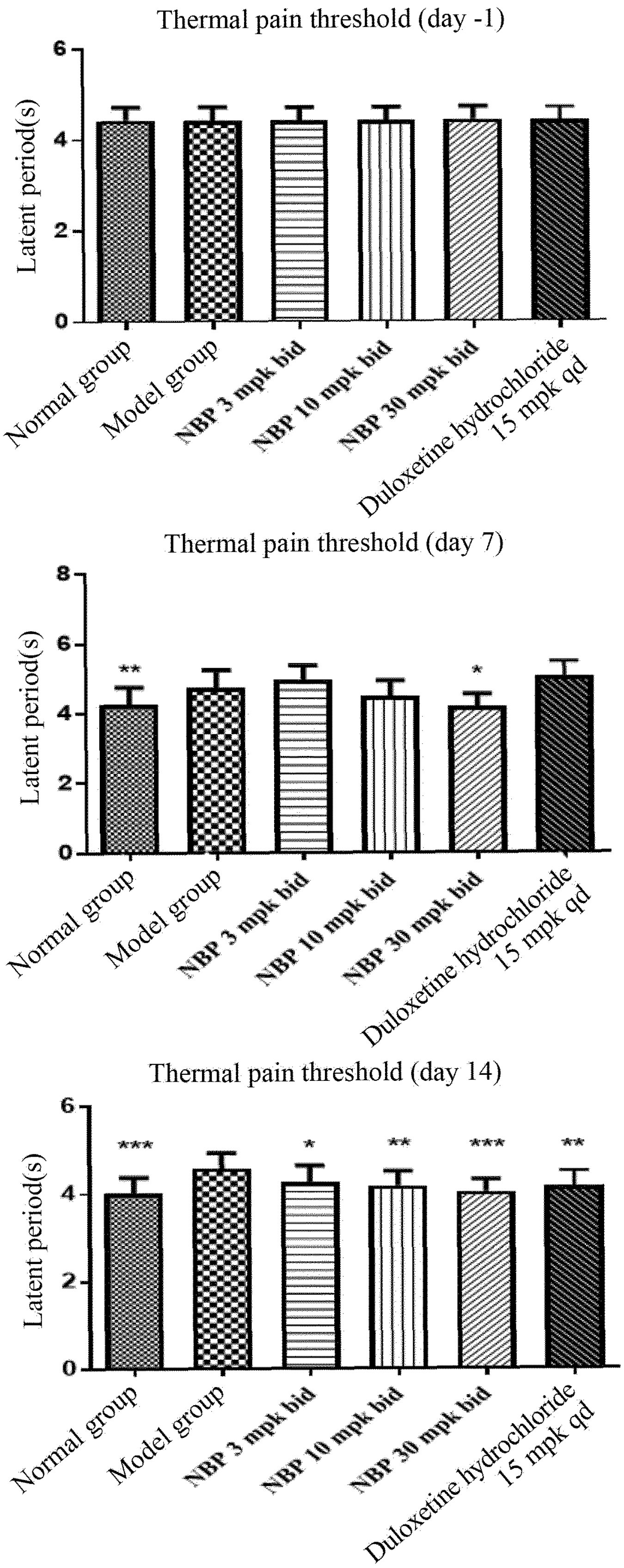
Thermal pain threshold (day -1)
Latent period(s)
6
4
2
0
Normal group
Model group
NBP 3 mpk bid
NBP 10 mpk bid
NBP 30 mpk bid
Duloxetine hydrochloride 15 mpk qd
Thermal pain threshold (day 7)
Latent period(s)
8
6
4
2
0
**
*
Normal group
Model group
NBP 3 mpk bid
NBP 10 mpk bid
NBP 30 mpk bid
Duloxetine hydrochloride 15 mpk qd
Thermal pain threshold (day 14)
Latent period(s)
6
4
2
0
***
*
**
***
**
Normal group
Model group
NBP 3 mpk bid
NBP 10 mpk bid
NBP 30 mpk bid
Duloxetine hydrochloride 15 mpk qd

Fig. 7

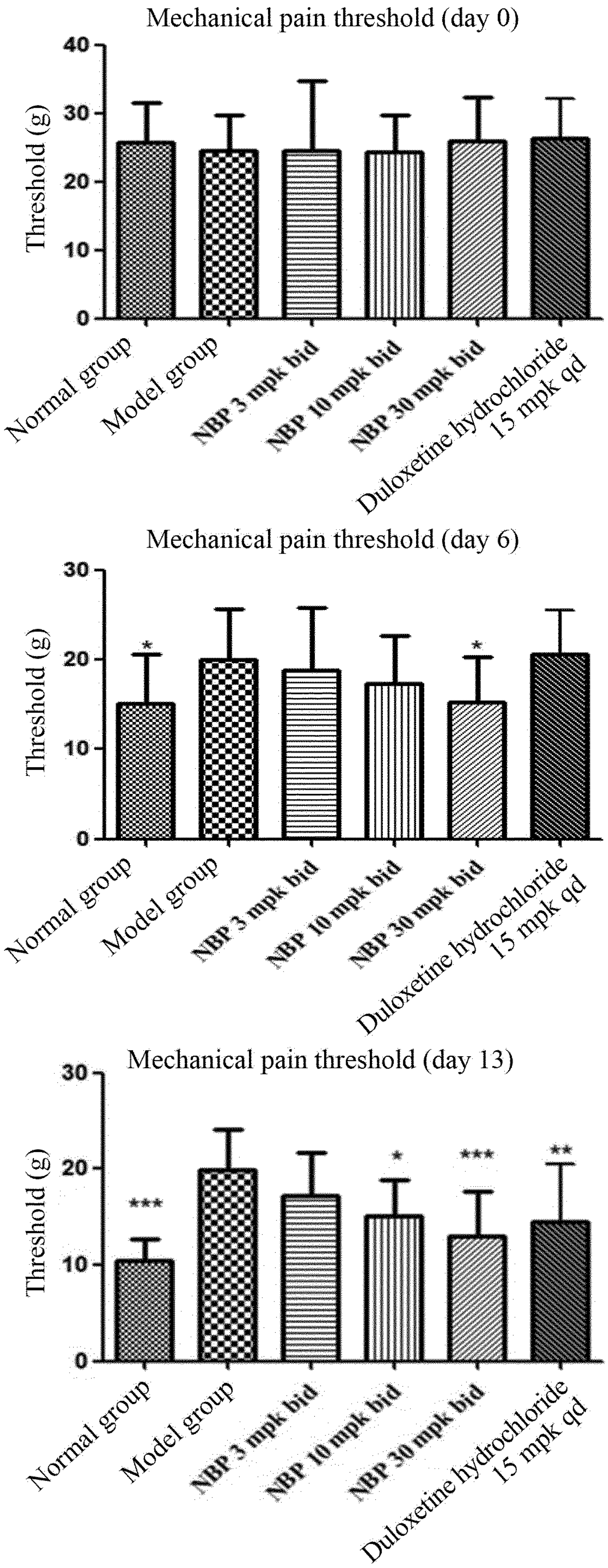
Mechanical pain threshold (day 0)
Threshold (g)
40
30
20
10
0
Normal group
Model group
NBP 3 mpk bid
NBP 10 mpk bid
NBP 30 mpk bid
Duloxetine hydrochloride 15 mpk qd
Mechanical pain threshold (day 6)
Threshold (g)
30
20
10
0
*
*
Normal group
Model group
NBP 3 mpk bid
NBP 10 mpk bid
NBP 30 mpk bid
Duloxetine hydrochloride 15 mpk qd
Mechanical pain threshold (day 13)
Threshold (g)
30
20
10
0
***
*
***
**
Normal group
Model group
NBP 3 mpk bid
NBP 10 mpk bid
NBP 30 mpk bid
Duloxetine hydrochloride 15 mpk qd

Fig. 8

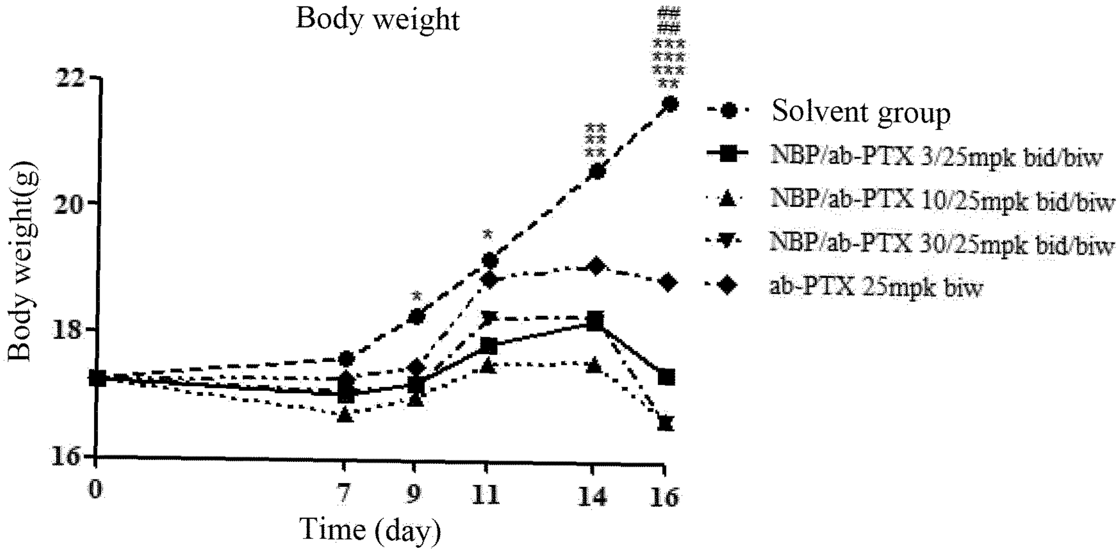
Body weight
Body weight(g)
22
20
18
16
0
7
9
11
14
16
Time (day)
Solvent group
NBP/ab-PTX 3/25mpk bid/biw
NBP/ab-PTX 10/25mpk bid/biw
NBP/ab-PTX 30/25mpk bid/biw
ab-PTX 25mpk biw

Fig. 9

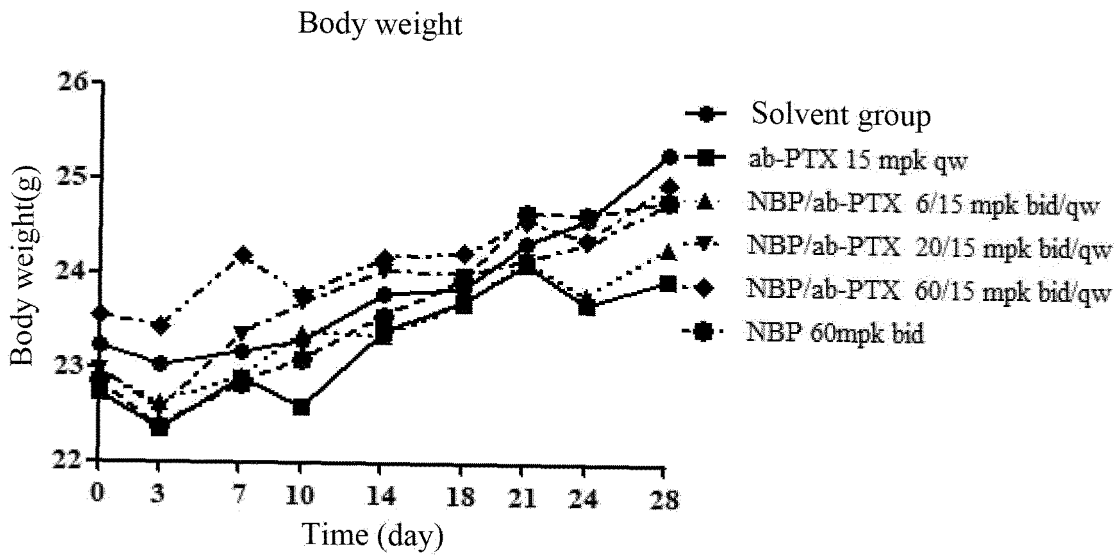
Body weight
Body weight(g)
26
25
24
23
22
0
3
7
10
14
18
21
24
28
Time (day)
Solvent group
ab-PTX 15 mpk qw
NBP/ab-PTX 6/15 mpk bid/qw
NBP/ab-PTX 20/15 mpk bid/qw
NBP/ab-PTX 60/15 mpk bid/qw
NBP 60mpk bid

Fig. 10

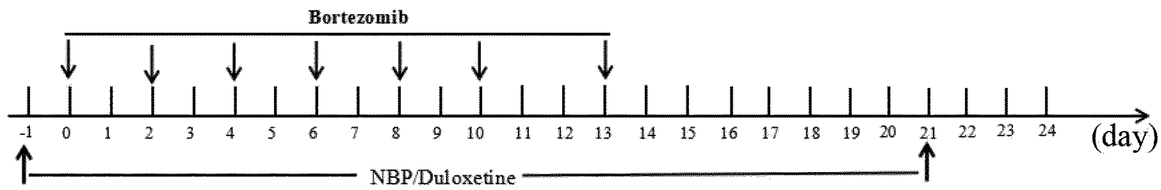
Bortezomib
-1 0 1 2 3 4 5 6 7 8 9 10 11 12 13 14 15 16 17 18 19 20 21 22 23 24
(day)
NBP/Duloxetine

Fig. 11

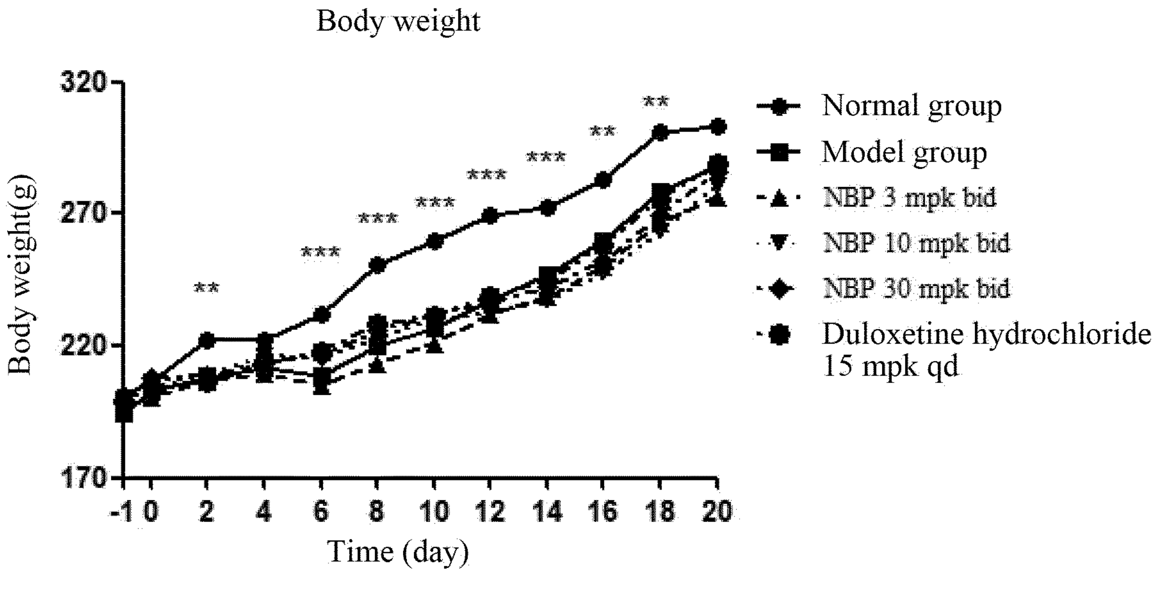
Body weight
Body weight(g)
320
270
220
170
-1 0 2 4 6 8 10 12 14 16 18 20
Time (day)
**
***
***
***
***
***
**
**
Normal group
Model group
NBP 3 mpk bid
NBP 10 mpk bid
NBP 30 mpk bid
Duloxetine hydrochloride 15 mpk qd

Fig. 12

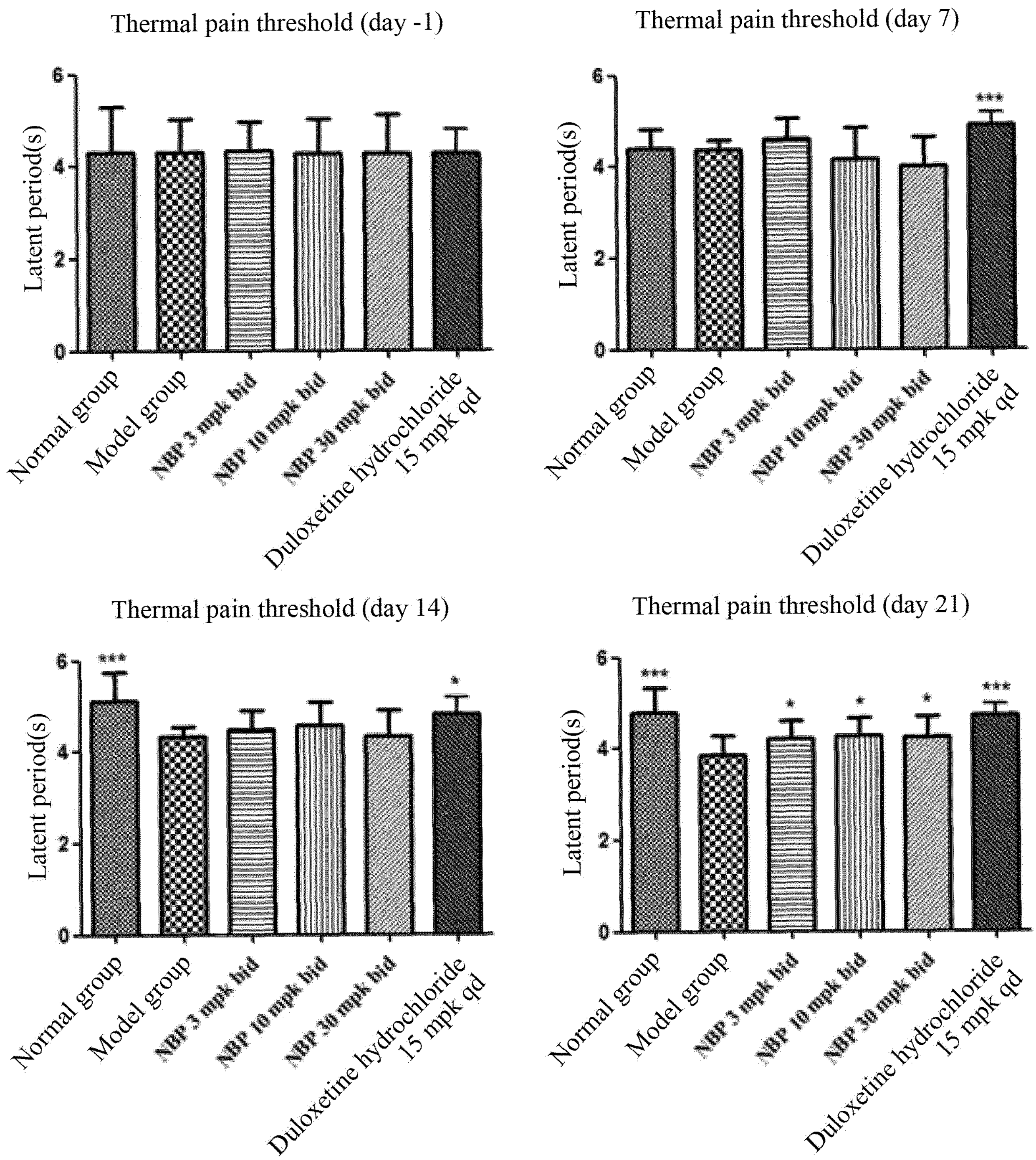
Thermal pain threshold (day -1)
Thermal pain threshold (day 7)
Thermal pain threshold (day 14)
Thermal pain threshold (day 21)
Latent period(s)
0
2
4
6
Normal group
Model group
NBP 3 mpk bid
NBP 10 mpk bid
NBP 30 mpk bid
Duloxetine hydrochloride 15 mpk qd
***
*

Fig. 13

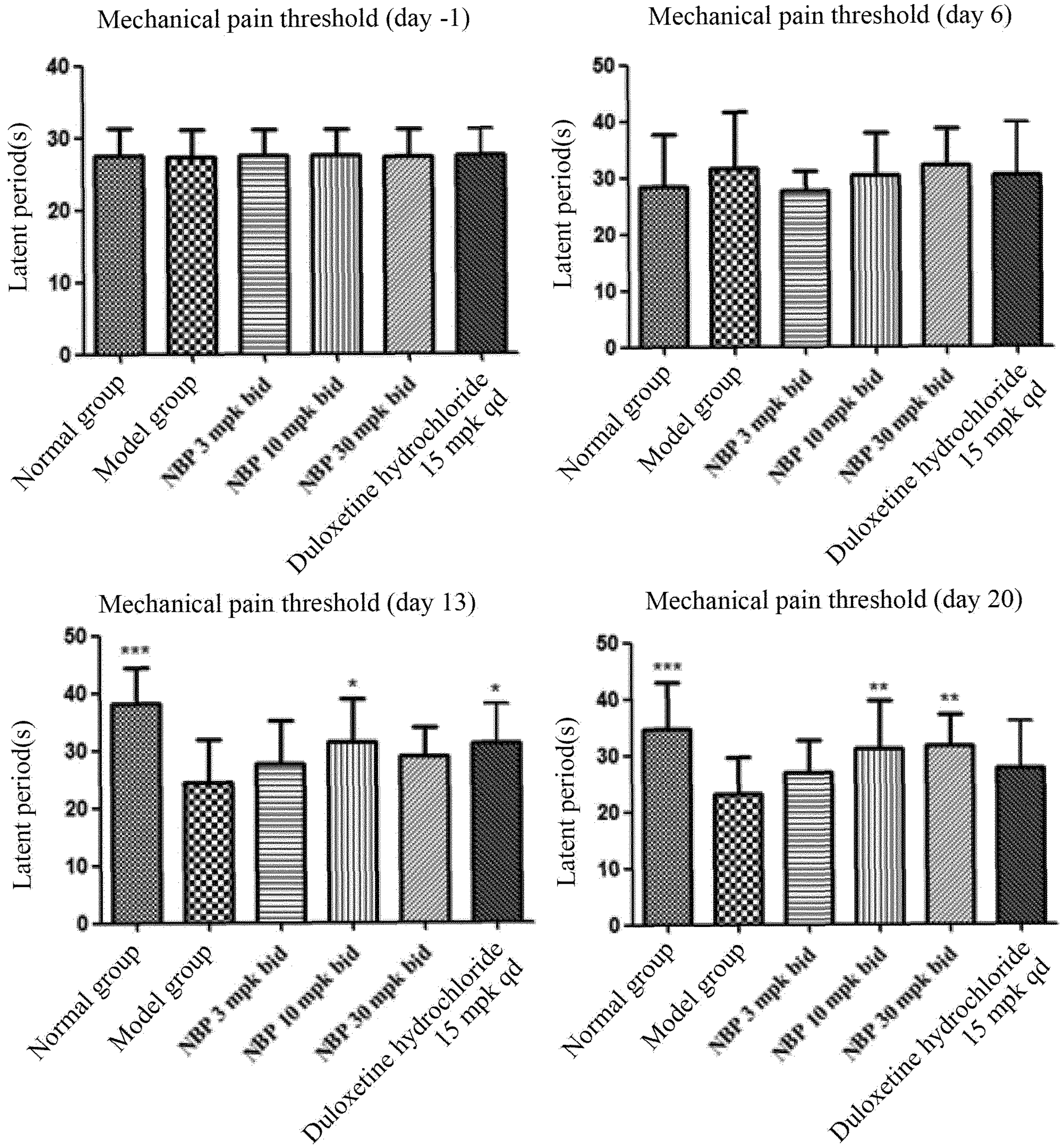
Mechanical pain threshold (day -1)
Latent period(s)
0
10
20
30
40
Normal group
Model group
NBP 3 mpk bid
NBP 10 mpk bid
NBP 30 mpk bid
Duloxetine hydrochloride 15 mpk qd
Mechanical pain threshold (day 6)
Latent period(s)
0
10
20
30
40
50
Normal group
Model group
NBP 3 mpk bid
NBP 10 mpk bid
NBP 30 mpk bid
Duloxetine hydrochloride 15 mpk qd
Mechanical pain threshold (day 13)
Latent period(s)
0
10
20
30
40
50
***
*
*
Normal group
Model group
NBP 3 mpk bid
NBP 10 mpk bid
NBP 30 mpk bid
Duloxetine hydrochloride 15 mpk qd
Mechanical pain threshold (day 20)
Latent period(s)
0
10
20
30
40
50
***
**
**
Normal group
Model group
NBP 3 mpk bid
NBP 10 mpk bid
NBP 30 mpk bid
Duloxetine hydrochloride 15 mpk qd

Fig. 14

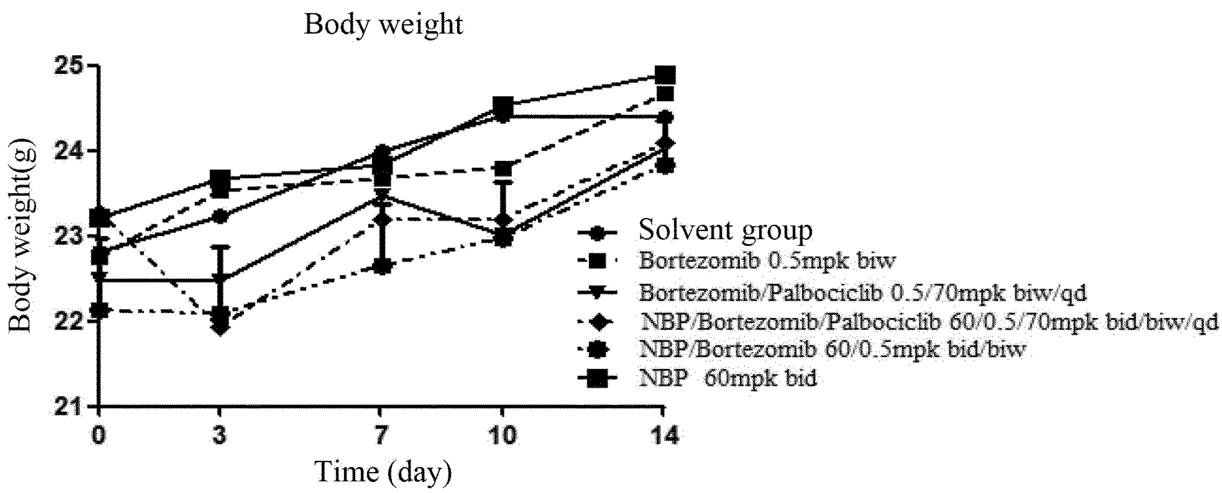
Body weight
Body weight(g)
25
24
23
22
21
0
3
7
10
14
Time (day)
Solvent group
Bortezomib 0.5mpk biw
Bortezomib/Palbociclib 0.5/70mpk biw/qd
NBP/Bortezomib/Palbociclib 60/0.5/70mpk bid/biw/qd
NBP/Bortezomib 60/0.5mpk bid/biw
NBP 60mpk bid

Fig. 15

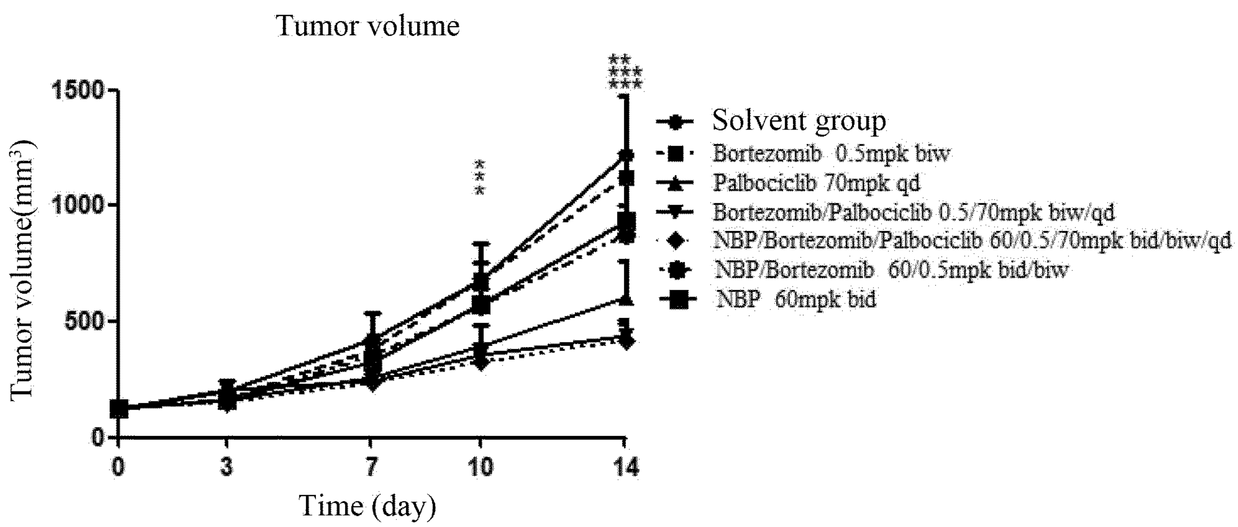
Tumor volume
Tumor volume(mm³)
1500
1000
500
0
0
3
7
10
14
Time (day)
Solvent group
Bortezomib 0.5mpk biw
Palbociclib 70mpk qd
Bortezomib/Palbociclib 0.5/70mpk biw/qd
NBP/Bortezomib/Palbociclib 60/0.5/70mpk bid/biw/qd
NBP/Bortezomib 60/0.5mpk bid/biw
NBP 60mpk bid

Fig. 16

**INTERNATIONAL SEARCH REPORT**

International application No.
**PCT/CN2021/081817**

**A. CLASSIFICATION OF SUBJECT MATTER**

A61K 31/365(2006.01)i; A61P 25/02(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CJFD; VEN; CNABS; CNKI; CNTXT; SIPOABS; USTXT; EPTXT; WOTXT; STN; Bing: 石药, 芹菜甲素, 癌, 丁基苯酞, 周围神经, 痛, 疼, 紫杉醇, 肿瘤, 化疗, 丁苯酞, NBP, Neuropathic pain, Peripheral Neuropath, Butylphthalide, Chemotherapy, 6066-49-5

**C. DOCUMENTS CONSIDERED TO BE RELEVANT**

| Category* | Citation of document, with indication, where appropriate, of the relevant passages | Relevant to claim No. |
|---|---|---|
| PX | "NBP in Women with Metastatic Breast Cancer to Prevent Nab-paclitaxel Induced Toxic Neuropathy" *https://clinicaltrials.gov/ct2/show/NCT04675450,* 03 March 2021 (2021-03-03), pp. 1-8 | 1-14 |
| X | 王满良 (WANG, Manliang). "丁苯酞软胶囊治疗糖尿病周围神经病变30例临床观察 (Investigation of Butylphthalide soft Capsules on Diabetic Peripheral Neuropathy)" *河北中医 (Hebei Journal of Traditional Chinese Medicine),* Vol. 35, No. 08, 31 August 2013 (2013-08-31), pages 1209-1211, abstract, section 1.3.1 | 1, 3-12, 14 |
| X | 赵玉武 等 (ZHAO, Yuwu et al.). "丁基苯酞治疗糖尿病性周围神经病的疗效观察 (Clinical Study on Efficacy of Butylphthalide on Diabetic Peripheral Neuropathy)" *中国神经免疫学和神经病学杂志 (Chinese Journal of Neuroimmunology and Neurology),* Vol. 16, No. 05, 30 September 2009 (2009-09-30), pp. 374-376 | 1, 3-12, 14 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 May 2021** | **23 June 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/081817**

**C. DOCUMENTS CONSIDERED TO BE RELEVANT**

| Category* | Citation of document, with indication, where appropriate, of the relevant passages | Relevant to claim No. |
|---|---|---|
| A | 王满良 (WANG, Manliang). "丁苯酞软胶囊治疗糖尿病周围神经病变30例临床观察 (Investigation of Butylphthalide soft Capsules on Diabetic Peripheral Neuropathy)" *河北中医 (Hebei Journal of Traditional Chinese Medicine),* Vol. 35, No. 08, 31 August 2013 (2013-08-31), pp. 1209-1211 | 2, 13 |
| A | LIAO, Dehua et al. "Neuroprotective Effects of dl-3-n-Butylphthalide against Doxorubicin-Induced Neuroinflammation, Oxidative Stress, Endoplasmic Reticulum Stress, and Behavioral Changes" *Oxidative Medicine and Cellular Longevity,* Vol. no. 2018, 16 August 2018 (2018-08-16), pp. 1-13 | 1-14 |
| A | CN 103505409 A (CSPC ZHONGQI PHARMACEUTICAL TECHNOLOGY (SHIJIAZHUANG) CO., LTD. et al.) 15 January 2014 (2014-01-15) entire document | 1-14 |

**INTERNATIONAL SEARCH REPORT**
**Information on patent family members**

International application No.
**PCT/CN2021/081817**

| Patent document cited in search report | Publication date (day/month/year) | Patent family member(s) | Publication date (day/month/year) |
|---|---|---|---|
| CN 103505409 A | 15 January 2014 | CN 107970208 A | 01 May 2018 |
| | | CN 107970208 B | 19 January 2021 |
| | | CN 103505409 B | 26 December 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010202588
- CN 202010790778
- CN 202011233563
- WO 2019242765 A1
- CN 1689563 A
- CN 102503919 A
- CN 101289438 A
- CN 1382682 A
- CN 1523003 A
- CN 104974050 A
- CN 108084233 A
- CN 109503548 A
- CN 108715579 A
- CN 106214674 A
- CN 101029037 A
- CN 101402565 A
- CN 104086399 A


### Non-patent literature cited in the description


- **SERETNY M ; CURRIE GL ; SENA ES et al.** Incidence, prevalence, and predictors of chemotherapy-induced peripheral neuropathy: a systemic review and meta-analysis. *Pain,* 2014, vol. 155, 2461-2470
- **GADGIL S ; ERGUN M ; VAN DEN HEUVAL SA et al.** A systematic summary and comparison of animal models for chemotherapy induced peripheral neuropathy (CIPN). *PLOS ONE,* 2019, https://doi.org/10.1371/journal.pone.0221787
- **EWERTZ M ; QVORTRUP C ; ECKHOFF L.** Chemotherapy-induced peripheral neuropathy in patients treated with taxanes and platinum derivatives. *ActaOncologica,* 2015, vol. 54, 587-591
- **PARK SB ; GOLDSTEIN D ; KRISHNAN AV et al.** Chemotherapy-induced peripheral neurotoxicity: A critical analysis. *CA Cancer J Clin,* 2013, vol. 63, 419-437
- **BKL D ; DE SOUZA S M ; COSTA-LIMA C et al.** Thalidomide for the treatment of gastrointestinal bleeding due to angiodysplasia in a patient with Glanzmann's thrombasthenia [J. *Hematol Rep,* 2017, vol. 9 (2), 6961
- **RUI GUI et al.** *Medical Recapitulate,* June 2012, vol. 18 (11), 1739-1742
- **BUROTTO M ; EDGERLY M ; VELARDE M et al.** A phase II multi-center study of bevacizumab in combination with ixabepilone in subjects with advanced renal cell carcinoma [J. *Oncologist,* 2017, vol. 22 (8), 888-e84
- **HERSHMAN DL ; LACCHETTI C ; DWORKIN RH et al.** Prevention and management of chemotherapy-induced peripheral neuropathy in survivors of adult cancers: American society of clinical oncology clinical practice guideline. *J Clin Oncol,* 2014, vol. 32, 1941-67
- **KOLB N ; BURNS T.** Clinical Research in chemotherapy-induced peripheral neuropathy. *Neurology,* 2018, vol. 91, 379-380